# EUROPÄISCHE PATENTANMELDUNG

(11) **EP 4 631 555 A1**
(43) Veröffentlichungstag der Anmeldung: **15.10.2025**
(21) Anmeldenummer: 25168724.0
(22) Anmeldetag: 07.04.2025
(51) Int. Cl.: A61M 16/00, A61M 16/01, A61M 16/20

(54) **FLUIDFORTLEITUNGSSYSTEM UND VERFAHREN ZUM FÖRDERN EINES FLUIDS**

(30) Priorität: 12.04.2024 DE 102024110226
(71) Anmelder: Drägerwerk AG & Co. KGaA, 23558 Lübeck (DE)
(72) Erfinder: Heesch, Ralf, 23558 Lübeck (DE); Schmid, Robert, 23558 Lübeck (DE)

(57) **Zusammenfassung**

Die Erfindung betrifft ein Fortleitungssystem und ein Verfahren zum Fördern eines Fluids. In einem Förderzustand des Fortleitungssystems fördert eine Ansaug-Anordnung (200) das Fluid von einer Quelle zu einer Senke. In einem Ruhezustand ist das Fördern von Fluid unterbunden oder unterbrochen. Ein Stecker (15) lässt sich in eine Steckdose (16) einstecken, und bei eingestecktem Stecker ist eine Fluidverbindung zwischen der Quelle und der Senke hergestellt. In einer Förder-Schaltstellung versetzt ein Schaltelement (38.1, 38.2) das Fortleitungssystem in den Förderzustand, in einer Ruhe-Schaltstellung in den Ruhezustand. Ein als passives Bauteil ausgestaltetes Rückstellelement (39.1, 39.2) hält in einem Ruhezustand das Schaltelement in der Förder-Schaltstellung. Ein eingeschaltetes Stellglied (42.1, 42.2) vermag das Schaltelement gegen eine rückstellende Kraft des Rückstellelements in die Ruhe-Schaltstellung zu schalten. Eine signalverarbeitende Steuereinheit vermag ein Ereignis zu detektieren, dass aus der Quelle kein Fluid austritt. Falls dieses Ereignis positiv detektiert ist, schaltet die Steuereinheit das Stellglied ein.

## Beschreibung

Die Erfindung betrifft ein Fortleitungssystem für ein Fluid, wobei das Fluid insbesondere ein Gasgemisch mit einem Anästhesiegas ist. Weiterhin betrifft die Erfindung ein Verfahren zum Fördern eines Fluids von einer Quelle zu einer Senke.

In einer Anwendung ist die Quelle ein Beatmungsgerät für eine künstliche Beatmung eines Patienten, wobei der Patient mit einem Gasgemisch versorgt wird. Das Gasgemisch umfassend Sauerstoff und mindestens ein Narkosemittel, optional zusätzlich ein Medikament. Die Senke ist ein Aufnahmesystem für überschüssiges Gasgemisch, wobei das aufgenommene Gasgemisch in der Regel Narkosemittel umfasst.

In DE 10 2012 109 866 B4 wird ein Anästhesiegas-Fortleitungssystem beschrieben. Dieses System saugt mittels Unterdruck überschüssiges Anästhesiegas aus einem Anästhesiegerät ab und fördert es in ein stationäres Aufnahmenetz eines Gebäudes. Der Unterdruck wird nur dann erzeugt, wenn detektiert worden ist, dass überschüssiges Gas tatsächlich aufgetreten ist oder die Möglichkeit des Auftritts besteht. Ein Stecker des Systems ist über einen Schlauch mit dem Anästhesiegerät verbunden und lässt sich in eine korrespondierende Steckdose in einer Wand einstecken. Ein Antrieb 6 vermag einen Stößel 4 im Stecker vor- und zurückzubewegen. Eine Feder 7 ist bestrebt, den Stößel 4 in eine Position zu bringen, in der der Stößel 4 aus der Spitze 1 des Steckers herausragt. Falls der Stößel 4 aus der Spitze 1 herausragt und der Stecker in die Steckdose eingesteckt ist, ist eine Fluidverbindung zwischen dem Anästhesiegerät und dem Aufnahmenetz hergestellt.

Der Erfindung liegt die Aufgabe zugrunde, ein Fluidfortleitungssystem und ein Verfahren zum Fördern eines Fluids bereitzustellen, welche eine höhere Betriebssicherheit als bekannte Fluidfortleitungssysteme und Verfahren aufweisen sollen.

Die Aufgabe wird durch ein Fluidfortleitungssystem mit den Merkmalen des Anspruchs 1 und durch ein Verfahren mit den Merkmalen des Anspruchs 13 gelöst. Vorteilhafte Ausgestaltungen sind in den Unteransprüchen angegeben. Vorteilhafte Ausgestaltungen des erfindungsgemäßen Fortleitungssystems sind, soweit sinnvoll, auch vorteilhafte Ausgestaltungen des erfindungsgemäßen Verfahrens und umgekehrt.

Das erfindungsgemäße Fortleitungssystem ist dazu ausgestaltet, ein Fluid fortzuleiten und hierbei von einer Quelle zu einer Senke zu fördern. Das Fluid ist bevorzugt ein Gas oder Gasgemisch, insbesondere ein Gasgemisch, welches von einem künstlich beatmeten Patienten ausgeatmet wird und welches Narkosemittel und / oder ein Medikament enthalten kann.

Das erfindungsgemäße Fortleitungssystem umfasst einen Stecker und eine Steckdose. Falls der Stecker in die Steckdose eingesteckt ist, so ist eine Fluidverbindung zwischen einer Quelle und einer Senke hergestellt oder lässt sich herstellen. Durch diese Fluidverbindung hindurch kann ein Fluid von der Quelle zur Senke fließen. Diese Fluidverbindung ist wenigstens dann unterbrochen, wenn der Stecker nicht in die Steckdose eingesteckt ist. Auch bei eingestecktem Stecker kann die Fluidverbindung zeitweise unterbrochen sein.

Das Fortleitungssystem lässt sich in einen Förderzustand und in einen Ruhezustand versetzen. Im Förderzustand ist die Fluidverbindung hergestellt. Eine Ansaug-Anordnung vermag dann, wenn das Fortleitungssystem im Förderzustand ist, Fluid von der Quelle zur Senke zu fördern. Die Ansaug-Anordnung saugt insbesondere das Fluid aus der Quelle und fördert das Fluid zur Senke. Im Ruhezustand ist die Fluidverbindung unterbrochen, und / oder die Ansaug-Anordnung ist ausgeschaltet. Möglich ist, dass im Ruhezustand der Stecker in die Steckdose eingesteckt und die Ansaug-Anordnung ausgeschaltet ist.

Ein Schaltelement des Fortleitungssystems lässt sich in eine Förder-Schaltstellung und in eine Ruhe-Schaltstellung schalten. Das Schaltelement in der Förder-Schaltstellung versetzt das Fortleitungssystem in den Förderzustand und hält es in diesem. Das Schaltelement in der Ruhe-Schaltstellung versetzt das Fortleitungssystem in den Ruhezustand und hält es in diesem.

Ein Rückstellelement des Fortleitungssystems ist als mechanisches Bauteil ausgestaltet und weist einen Ruhezustand auf. Unter einem "mechanischen Bauteil" wird ein Bauteil verstanden, welches eine Bewegung auszuführen vermag und hierfür nicht mit elektrischer oder pneumatischer oder hydraulischer Energie versorgt zu werden braucht. Bevorzugt umfasst das Rückstellelement mindestens eine mechanische oder pneumatische Feder.

Im Ruhezustand hält das Rückstellelement das Schaltelement in der Förder-Schaltstellung. Falls das Rückstellelement aus dem Ruhezustand ausgelenkt wird, so übt das Rückstellelement eine rückstellende Kraft aus, und die rückstellende Kraft ist bestrebt, das Rückstellelement wieder in den Ruhezustand zu überführen und im Ruhezustand zu halten. Die Rückstellkraft wird insbesondere mechanisch und / oder pneumatisch und / oder hydraulisch erzeugt. Das Rückstellelement ist so mit dem Schaltelement verbunden, dass die rückstellende Kraft des Rückstellelements bestrebt ist, das Schaltelement in die Förder-Schaltstellung zu schalten und / oder in dieser zu halten.

Ein Stellglied des Fortleitungssystems lässt sich durch eine entsprechende Ansteuerung einschalten und ausschalten. Das eingeschaltete Stellglied vermag das Schaltelement gegen die rückstellende Kraft des Rückstellelements aus der Förder-Schaltstellung in die Ruhe-Schaltstellung zu schalten und in dieser zu halten und dadurch das Fortleitungssystem in den Ruhezustand zu überführen. Bei ausgeschaltetem Stellglied hält das Rückstellelement das Schaltelement in der Förder-Schaltstellung. Das Ausschalten des Stellglieds bewirkt, dass das Rückstellelement das Schaltelement in die Förder-Schaltstellung bewegt.

Eine signalverarbeitende Steuereinheit vermag das Stellglied anzusteuern und dadurch einzuschalten und bevorzugt das Stellglied auch wieder auszuschalten. Die Steuereinheit vermag das Fortleitungssystem darauf zu überwachen, ob mindestens ein vorgegebenes mögliches Ereignis tatsächlich aufgetreten ist. Das oder jedes mögliche Ereignis bedeutet oder hat zur Folge, dass aus der Quelle kein Fluid austritt.

Die Steuereinheit ist wie folgt ausgestaltet: Falls die Steuereinheit positiv das oder mindestens ein solches Ereignis detektiert hat, so schaltet die Steuereinheit das Stellglied ein. Dass ein mögliches Ereignis "positiv detektiert" ist, bedeutet folgendes: Die Steuereinheit hat mit ausreichender Sicherheit festgestellt, dass dieses Ereignis tatsächlich eingetreten ist. Im Zweifelsfall schaltet also die Steuereinheit nicht das Stellglied ein.

Das erfindungsgemäße Verfahren wird unter Verwendung eines derartigen Fortleitungssystems durchgeführt. Das Verfahren umfasst die folgenden Schritte:
- Der Stecker ist oder wird in die Steckdose gesteckt.
- Das Schaltelement ist oder wird in die Förder-Schaltstellung geschaltet.
- Das Schaltelement in der Förder-Schaltstellung versetzt oder hält das Fortleitungssystem im Förderzustand.

Während das Fortleitungssystem im Förderzustand ist, so werden die folgenden Schritte durchgeführt:
- Die Fluidverbindung zwischen der Quelle und der Senke ist oder wird hergestellt. Diese Fluidverbindung führt durch den Stecker und durch die Steckdose hindurch.
- Die Ansaug-Anordnung fördert das Fluid durch die Fluidverbindung hindurch von der Quelle zur Senke, bevorzugt durch Ansaugen.
- Das Stellglied ist ausgeschaltet.
- Das Rückstellelement ist im Ruhezustand und hält das Schaltelement in der Förder-Schaltstellung.
- Bei einer Auslenkung aus dem Ruhezustand übt das Rückstellelement eine rückstellende Kraft aus. Die rückstellende Kraft ist bestrebt, das Schaltelement in die Förder-Schaltstellung zu schalten und / oder das Schaltelement in der Förder-Schaltstellung zu halten.
- Die Steuereinheit überwacht das Fortleitungssystem und / oder die Quelle auf mindestens ein vorgegebenes mögliches Ereignis, bei dem aus der Quelle kein Fluid austritt.

Die Durchführung der nachfolgend beschriebenen weiteren Schritte wird ausgelöst,
- während der Stecker in die Steckdose eingesteckt ist und
- falls die Steuereinheit detektiert hat, dass ein vorgegebenes mögliches Ereignis tatsächlich eingetreten ist.

Falls dieses Ereignis positiv detektiert ist, steht fest, dass aus der Quelle kein Fluid austritt und daher auch kein Fluid zur Senke gefördert zu werden braucht.

In dieser Situation werden folgende Schritte durchgeführt:
- Die Steuereinheit schaltet das Stellglied ein.
- Das eingeschaltete Stellglied schaltet das Schaltelement in die Ruhe-Schaltstellung, und zwar gegen die rückstellende Kraft des Rückstellelements, und hält es in dieser.
- In der Ruhe-Schaltstellung bewirkt das Schaltelement, dass das Fortleitungssystem in den Ruhezustand versetzt und im Ruhezustand gehalten wird. Im Ruhezustand des Fortleitungssystems ist das Fördern von Fluid durch die Fluidverbindung hindurch unterbrochen und / oder unterbunden.

Während das Fortleitungssystem im Förderzustand ist, fördert das Fortleitungssystem Fluid aus der Quelle zur Senke und verhindert dadurch in vielen Fällen, dass sich in der Quelle zu viel Fluid ansammelt und dadurch beispielsweise ein unerwünschter Überdruck in der Quelle entsteht.

In vielen Fällen liegt ein sicherer Zustand dann vor, wenn das Fortleitungssystem im Förderzustand ist und Fluid von der Quelle zur Senke fördert. Das Rückstellelement ist bestrebt, das Schaltelement in der Förder-Schaltstellung zu halten und dadurch sicherzustellen, dass das Fortleitungssystem im Förderzustand ist und bleibt. Weil das Rückstellelement als mechanisches Bauteil ausgestaltet ist und in seinem Ruhezustand das Schaltelement in der Förder-Schaltstellung hält, verbleibt das Fortleitungssystem auch dann im Förderzustand, wenn eine elektrische oder pneumatische oder hydraulische Versorgung des Schaltelements ausgefallen oder unterbrochen oder das Schaltelement selbst ausgefallen oder ausgeschaltet ist. Das Fortleitungssystem ist auch dann im Förderzustand, wenn das Stellglied oder eine Versorgung des Stellglieds ausgefallen sind. Die Erfindung erhöht dadurch die Zuverlässigkeit, dass tatsächlich der sichere Zustand hergestellt ist.

Falls hingegen kein überschüssiges Fluid in der Quelle auftritt, so ist es auch nicht erforderlich, Fluid von der Quelle zur Senke zu fördern. In dieser Situation kann das Fortleitungssystem sich im Ruhezustand befinden und verbraucht dann weniger Energie als im Förderzustand. Diese Ausgestaltung spart elektrische Energie ein verglichen mit einem Zustand, bei dem das Fortleitungssystem dauerhaft im Förderzustand ist.

Die Erfindung bewirkt folgendes: Falls positiv festgestellt worden ist, dass kein überschüssiges Fluid in der Quelle auftritt und es daher ein sicherer Zustand ist, dass das Fortleitungssystem im Ruhezustand ist, so wird das Stellglied eingeschaltet und dadurch das Schaltelement in den Ruhe-Schaltzustand überführt und in diesem gehalten. Dies geschieht gegen die rückstellende Kraft des Rückstellelements. Im Zweifelsfall wird das Fortleitungssystem im Förderzustand gehalten oder in den Förderzustand geschaltet. Dieser Zweifelsfall liegt vor, wenn weder mit ausreichender Sicherheit festgestellt werden kann, dass kein überschüssiges Fluid aufgetreten ist, noch mit ausreichender Sicherheit festgestellt werden kann, dass überschüssiges Fluid vorliegt und abgeführt werden muss.

Wie bereits dargelegt, ist in der Regel der sichere Zustand der, dass das Fortleitungssystem im Förderzustand ist. Im Förderzustand ist in der Regel das Stellglied ausgeschaltet und verbraucht keine elektrische Energie und braucht auch nicht mit einem hydraulischen oder pneumatischen Fluid versorgt zu werden.

Dank der Erfindung ist es nicht erforderlich, dass das Stellglied das Schaltelement in beide Richtungen zu bewegen vermag. Vielmehr ist es ausreichend, dass das Stellglied das Schaltelement gegen die rückstellende Kraft in die Ruhe-Schaltstellung zu überführen vermag. Bei ausgeschaltetem Stellglied überführt das Rückstellelement das Schaltelement in die Förder-Schaltstellung und hält es in dieser. Weil in der Regel die Förder-Schaltstellung des Stellglieds zu einem sicheren Zustand führt, ist es häufig nicht erforderlich, dass das Stellglied das Schaltelement rasch in die Ruhe-Schaltstellung zu überführen vermag. Das Stellglied braucht daher nur so viel Kraft aufzubringen, dass die rückstellende Kraft des Rückstellelements überwunden wird.

Erfindungsgemäß ist mindestens ein mögliches detektierbares Ereignis vorgegeben, bei dem feststeht, dass aus der Quelle kein Fluid austritt. Beispielsweise steht fest, dass aus einem Gerät, welches die Quelle umfasst, kein Fluid austritt. Ein Indiz hierfür kann sein, dass dieses Gerät keinen elektrischen Strom aufnimmt oder verbraucht. Die Steuereinheit vermag das oder jedes vorgegebene Ereignis zu detektieren oder festzustellen, dass kein solches Ereignis eingetreten ist. Verschiedene Ausgestaltungen sind möglich, welche derartigen Ereignisse vorgegeben sind werden können. Möglich ist auch, dass mindestens ein Ereignis vorgegeben ist, bei dem sicher oder wenigstens möglicherweise Fluid aus der Quelle austritt oder austreten kann.

In einer Ausgestaltung ist das oder ein detektierbares Ereignis das Ereignis, dass die Quelle - oder ein Gerät mit der Quelle - ausgeschaltet ist oder ausgeschaltet worden ist und nicht wieder eingeschaltet ist. In der Regel vermag eine ausgeschaltete Quelle kein Fluid auszustoßen. Die Steuereinheit vermag dieses Ereignis zu detektieren.

In einer Ausgestaltung vermag die Steuereinheit ein Indiz dafür zu detektieren, dass aus der Quelle aktuell tatsächlich Fluid austritt. Optional vermag die Steuereinheit zusätzlich ein Indiz dafür zu detektieren, dass die Quelle dazu betriebsbereit ist, Fluid auszustoßen. Falls die Steuereinheit ein solches Indiz detektiert hat, so bewirkt die Steuereinheit oder stellt sicher, dass das Stellglied ausgeschaltet wird oder ausgeschaltet ist. Diese Ausgestaltung bewirkt also, dass das Fortleitungssystem in einen sicheren Zustand, nämlich in den Förderzustand, überführt wird, wenn aus der Quelle Fluid austritt oder die Quelle wenigstens dafür bereit ist.

In einer Realisierungsform dieser Ausgestaltung vermag die Steuereinheit als Indiz dafür, dass aus der Quelle Fluid austritt und / oder die Quelle betriebsbereit ist, eines der folgenden drei Indizien zu detektieren:
- Die Quelle nimmt elektrische Energie auf oder verbraucht sie, beispielsweise für einen internen elektrischen Verbraucher. Die Energieaufnahme liegt oberhalb einer vorgegebenen unteren Schranke.
- Die Quelle gibt elektrische Energie ab, beispielsweise an einen angeschlossenen externen elektrischen Verbraucher. Die Energieabgabe liegt oberhalb einer vorgegebenen unteren Schranke.
- Ein Fluid fließt in die Quelle hinein.

In einer Ausgestaltung vermag die Steuereinheit ein Indiz für folgendes Ereignis zu detektieren: Eine Datenverbindung zwischen der Quelle oder einem Gerät, das die Quelle umfasst, einerseits und der Steuereinheit andererseits ist unterbrochen. Beispielsweise sendet die Steuereinheit eine Abfrage an das Gerät mit der Quelle, aber das Gerät antwortet nicht. Falls die Steuereinheit ein Indiz für eine unterbrochene Datenverbindung detektiert hat, so bewirkt die Steuereinheit folgendes: Der Schritt wird ausgelöst, dass das Stellglied ausgeschaltet wird. Oder sichergestellt wird, dass der Stellglied ausgeschaltet ist und bleibt. Das Rückstellelement schaltet das Schaltelement in die Förder-Schaltstellung. Durch diese Ausgestaltung wird bei einer unterbrochenen Datenverbindung ein sicherer Zustand hergestellt, nämlich dass das Fortleitungssystem im Förderzustand ist.

Erfindungsgemäß lässt das Fortleitungssystem sich in einen Förderzustand und in einen Ruhezustand versetzen. Im Förderzustand fördert die Ansaug-Anordnung Fluid von der Quelle zur Senke. Unterschiedliche Ausgestaltungen sind möglich, wie ein Ruhezustand des Fortleitungssystems bewirkt wird.

In einer Ausgestaltung lässt die Ansaug-Anordnung sich einschalten und ausschalten. Bei eingeschalteter Ansaug-Anordnung ist das Fortleitungssystem im Förderzustand, bei ausgeschalteter Ansaug-Anordnung im Ruhezustand. Das Schaltelement vermag die Ansaug-Anordnung wahlweise in einen eingeschalteten oder in einen ausgeschalteten Zustand zu versetzen und in diesem Zustand zu halten. Die Ausgestaltung, dass die Ansaug-Anordnung sich ausschalten lässt, spart elektrische Energie ein verglichen mit einer Ausgestaltung, bei der die Ansaug-Anordnung dauerhaft eingeschaltet ist. Bevorzugt ist die Ansaug-Anordnung einer bestimmten Quelle zugeordnet.

Erfindungsgemäß lässt der Stecker des Fortleitungssystems sich in die Steckdose stecken. In der Ausgestaltung, bei der die Ansaug-Anordnung sich einschalten und ausschalten lässt, ist der Stecker bevorzugt wie folgt ausgestaltet: Das Rückstellelement und das Stellglied sind Bestandteile des Steckers. Weiterhin umfasst der Stecker ein Betätigungselement. Das Betätigungselement ist relativ zu einem Gehäuse des Steckers beweglich gelagert. Falls der Stecker in die Steckdose eingesteckt ist, so ist das Betätigungselement auch relativ zur Steckdose beweglich.

Das Betätigungselement lässt sich in eine Betätigungsposition bewegen. Falls das Betätigungselement in der Betätigungsposition ist, betätigt das Betätigungselement das Schaltelement. Die Betätigung bewirkt, dass das Schaltelement in die Förder-Schaltstellung überführt wird. Das betätigte Schaltelement schaltet die Ansaug-Anordnung ein und / oder hält die Ansaug-Anordnung im eingeschalteten Zustand. Das betätigte Schaltelement bewirkt also, dass die Ansaug-Anordnung eingeschaltet ist und dadurch das Fortleitungssystem im Förderzustand ist.

Verschiedene Ausgestaltungen sind möglich, wie das Betätigungselement bewegt wird. Möglich ist, dass das Betätigungselement sich von außen ansteuern oder direkt von einem Menschen betätigen lässt. Nachfolgend wird eine Realisierungsform beschrieben, die keine Ansteuerung oder Betätigung von außen erfordert.

Erfindungsgemäß weist das Rückstellelement einen Ruhezustand auf. Falls das Rückstellelement aus dem Ruhezustand ausgelenkt wird, so übt das Rückstellelement eine rückstellende Kraft aus. In einer Realisierungsform hält das Rückstellelement im Ruhezustand das gerade beschriebene Betätigungselement in der Betätigungsposition. Das Rückstellelement im Ruhezustand bewirkt dadurch, dass das Fortleitungssystem im Förderzustand bleibt, und gewährleistet damit einen sicheren Zustand.

Erfindungsgemäß vermag die Steuereinheit das Stellglied einzuschalten. In der gerade beschriebenen Ausgestaltung vermag das eingeschaltete Stellglied das Betätigungselement aus der Betätigungsposition heraus zu bewegen, und zwar gegen die rückstellende Kraft des Rückstellelements. Das eingeschaltete Stellglied bewirkt dadurch, dass
- das Schaltelement in die Ruhe-Schaltstellung überführt,
- die Ansaug-Anordnung ausgeschaltet und
- dadurch das Fortleitungssystem in den Ruhezustand überführt und im Ruhezustand gehalten wird.

Erfindungsgemäß lässt sich dann, wenn der Stecker in die Steckdose eingesteckt ist, eine Fluidverbindung zwischen der Quelle und der Senke herstellen. In einer Ausgestaltung ist mindestens ein Schaltventil in der Fluidverbindung angeordnet, wobei die Fluidverbindung von der Quelle zur Senke führt. Das oder jedes Schaltventil gehört bei dieser Ausgestaltung zum Schaltelement. Das oder jedes Schaltventil lässt sich jeweils in eine Förder-Schaltstellung und in eine Ruhe-Schaltstellung schalten. Diese beiden Schaltstellungen sind die Schaltstellungen des erfindungsgemäßen Schaltelements. In der Förder-Schaltstellung ist das Schaltventil geöffnet und gibt die Fluidverbindung frei. In der Ruhe-Schaltstellung ist das Schaltventil geschlossen und unterbricht die Fluidverbindung.

Das Rückstellelement hält dann, wenn es im Ruhezustand ist, das Schaltventil in der Förder-Schaltstellung. Das eingeschaltete Stellglied vermag das Schaltventil in die Ruhe-Schaltstellung zu schalten, und zwar gegen die rückstellende Kraft des Rückstellelements, und in dieser zu halten. Wird der Stellglied wieder ausgeschaltet, so überführt das Rückstellelement das Schaltventil wieder in die Förder-Schaltstellung.

Die Ausgestaltung mit dem oder mindestens einem Schaltventil ermöglicht es, dass die Ansaug-Anordnung dauerhaft eingeschaltet ist. Eine dauerhaft eingeschaltete Ansaug-Anordnung vermag in vielen Fällen Fluid von verschiedenen Quellen zu fördern, insbesondere anzusaugen. Außerdem steht die Ansaug-Anordnung bei Bedarf sofort zur Verfügung und muss nicht erst hochgefahren werden. Möglich ist, eine solche dauerhaft eingeschaltete Ansaug-Anordnung mit einer eigenen Spannungsversorgungseinheit zu versehen, so dass die Ansaug-Anordnung auch bei einem Ausfall eines stationären Spannungsversorgungsnetzes noch betriebsbereit ist. Die Anordnung mit dem oder mindestens einen Schaltventil lässt sich auch mit einer Ansaug-Anordnung kombinieren, die sich einschalten und ausschalten lässt.

In einer Ausgestaltung ist die Quelle ein Bestandteil eines Geräts, bevorzugt eines medizinischen Geräts, insbesondere eines Beatmungsgeräts. Aus diesem Gerät wird das Fluid ausgestoßen oder tritt aus. Die Steuereinheit umfasst bevorzugt ein eigenes Gehäuse. Die Steuereinheit ist räumlich beabstandet vom Gerät mit der Quelle angeordnet. Bevorzugt umfasst die Steuereinheit eine eigene Spannungsversorgungseinheit und / oder ein eigenes Koppelelement, welches sich mit einem stationären Spannungsversorgungsnetz netzverbinden lässt. Die Ausgestaltung, dass die Steuereinheit räumlich beabstandet ist, erleichtert es, die Erfindung in ein bestehendes Fortleitungssystem zu integrieren. In vielen Fällen braucht das Gerät mit der Quelle nicht oder nur wenig verändert zu werden. Außerdem lässt die Steuereinheit sich leichter überwachen und austauschen.

Die Erfindung betrifft weiterhin ein System, welches ein erfindungsgemäßes Fortleitungssystem, eine Quelle für ein Fluid und eine Senke für das Fluid umfasst. Falls der Stecker des Fortleitungssystems in die Steckdose eingeschaltet ist, so ist eine Fluidverbindung zwischen der Quelle und der Senke des Systems hergestellt oder lässt sich herstellen. Die Ansaug-Anordnung vermag das Fluid von der Quelle durch den Stecker hindurch zur Senke zu fördern. Falls der Stecker nicht eingesteckt ist, so ist diese Fluidverbindung unterbrochen. Die gerade beschriebenen Ausgestaltungen und Vorteile des Fortleitungssystems gelten auch für dieses System.

In einer Ausgestaltung umfasst das System ein Beatmungsgerät. Das Beatmungsgerät ist zur künstlichen Beatmung eines Patienten ausgestaltet und vermag ein atembares Gasgemisch auszustoßen. Während das Beatmungsgerät die künstliche Beatmung durchführt, ist im und / oder am Körper des Patienten eine patientenseitige Koppeleinheit angeordnet. Die patientenseitige Koppeleinheit umfasst beispielsweise eine Atemmaske oder einen Tubus. Während der künstlichen Beatmung ist dauerhaft oder wenigstens zeitweise eine Fluidverbindung zwischen dem Beatmungsgerät und der patientenseitigen Koppeleinheit hergestellt. Das atembare Gasgemisch, welches das Beatmungsgerät ausstößt, fließt durch diese Fluidverbindung hindurch zur patientenseitigen Koppeleinheit. Der Patient kann dieses Gasgemisch einatmen.

Das erfindungsgemäße Fortleitungssystem vermag ein Fluid von der Quelle zur Senke zu fördern. Gemäß der gerade beschriebenen Ausgestaltung liegt die Quelle im Beatmungsgerät oder in der Fluidverbindung zwischen dem Beatmungsgerät und der patientenseitigen Koppeleinheit. Das Fluid, welches die Quelle bereitstellt, umfasst bevorzugt Gas, das der künstlich beatmete Patient ausgeatmet hat. Die Senke umfasst eine Aufnahme-Anordnung für das Fluid. Dank der Aufnahme-Anordnung wird verhindert, dass das ausgeatmete Gas in die Umgebung gelangt.

Möglich ist, dass wenigstens ein Teil der vom Patienten ausgeatmeten Luft in die Umgebung geleitet wird. In einer abweichenden Realisierungsform ist hingegen zwischen dem Beatmungsgerät und der patientenseitigen Koppeleinheit ein Beatmungskreislauf hergestellt. Diese Ausgestaltung verhindert, dass vom Patienten ausgeatmetes Gas in die Umgebung des Systems gerät. Dies ist insbesondere in folgender Situation wichtig: Das Gasgemisch, welches vom Beatmungsgerät ausgestoßen wird und zur patientenseitigen Koppeleinheit fließt, enthält mindestens ein Narkosemittel und optional ein Medikament. Dadurch ist der Patient narkotisiert oder wenigstens sediert. Das vom Patienten ausgeatmete Gas enthält daher ein Narkosemittel. Der Beatmungskreislauf verhindert, dass dieses Narkosemittel in die Umgebung des Systems gerät. Die Quelle für das Fluid liegt im Beatmungskreislauf. Das Fluid ist beispielsweise überschüssiges Gas aus dem Beatmungskreislauf.

Im Folgenden wird die Erfindung anhand eines Ausführungsbeispiels beschrieben. Hierbei zeigt
- Figur 1: schematisch eine erste Ausgestaltung der erfindungsgemäßen Beatmungs-Anordnung mit einer einschaltbaren und ausschaltbaren Unterdruckquelle;
- Figur 2: schematisch eine zweite Ausgestaltung der erfindungsgemäßen Beatmungs-Anordnung mit einem Schaltventil;
- Figur 3: den Stecker des Fluidfortleitungssystems und eine erste Ausgestaltung der einschaltbaren und ausschaltbaren Unterdruckquelle;
- Figur 4: den Stecker von Figur 3 und eine zweite Ausgestaltung der Unterdruckquelle;
- Figur 5: schematisch die Steuereinheit, welche das Stellglied des Steckers einschaltet und ausschaltet.

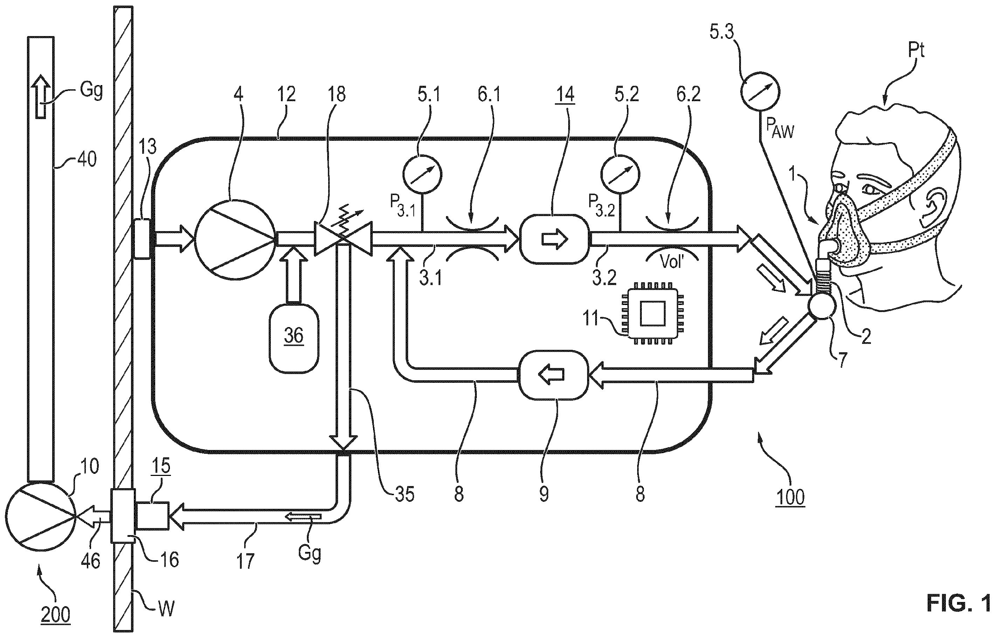

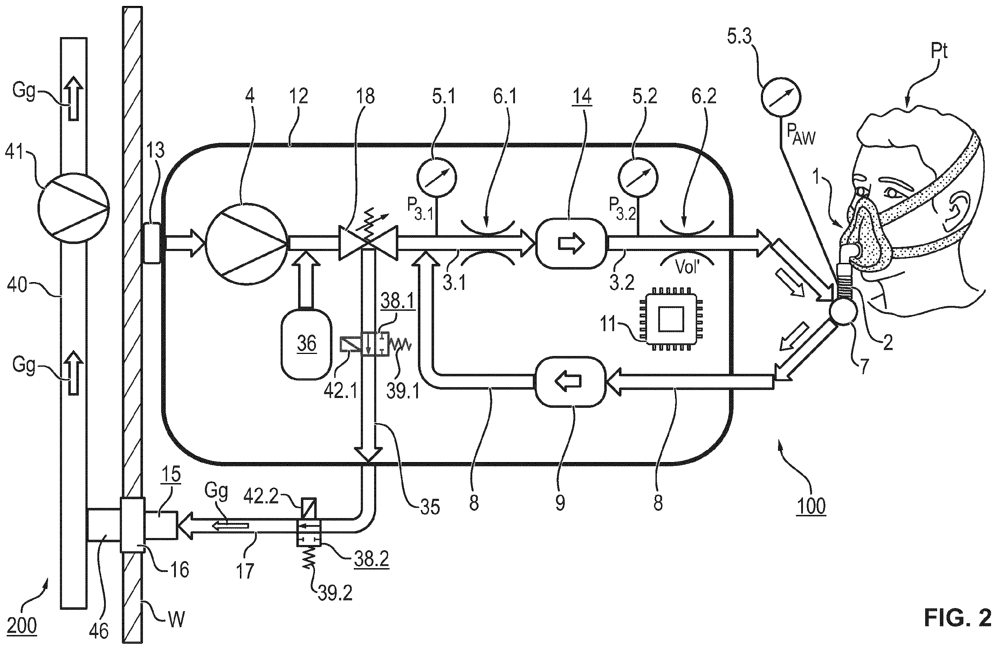

Figur 1 und Figur 2 zeigen schematisch eine bevorzugte Anwendung der Erfindung. Gleiche Bezugszeichen haben die gleichen Bedeutungen.

Ein Patient Pt wird künstlich beatmet. Am und / oder im Körper des Patienten Pt ist eine patientenseitige Koppeleinheit angebracht, im Ausführungsbeispiel eine Atemmaske 1 auf seinem Gesicht. Eine Inspirations-Fluidführungseinheit, beispielsweise ein Schlauch, umfasst zwei nachfolgend beschriebene Segmente 3.1 und 3.2 und verbindet ein schematisch gezeigtes Beatmungsgerät 12 mit einem Y-Stück 7. Die patientenseitige Koppeleinheit 1 steht über eine Fluidführungseinheit 2 in einer Fluidverbindung mit dem Y-Stück 7.

Im Ausführungsbeispiel ist der Patient Pt mit Hilfe eines Narkosemittels narkotisiert oder wenigstens sediert. Das Beatmungsgerät 12 umfasst einen schematisch gezeigten Versorgungsanschluss 13 für Atemluft und Sauerstoff und optional für Druckluft und / oder für ein Trägergas für Narkosemittel. Der Versorgungsanschluss 13 ist in eine Wand W eingelassen. Ein Narkosemittelverdunster 36 speist Narkosemittel in ein Trägergas ein und erzeugt dadurch einen Strom von gasförmigem Narkosemittel.

Das Beatmungsgerät 12 stößt ein atembares Gasgemisch aus, welches Sauerstoff und mindestens ein Narkosemittel umfasst. Bevorzugt führt das Beatmungsgerät 12 eine Abfolge von Beatmungshüben (ventilation strokes) aus und stößt in jedem Beatmungshub jeweils eine Menge des Gasgemischs aus. Das ausgestoßene Gasgemisch fließt durch die Inspirations-Fluidführungseinheit 3.1, 3.2 hindurch zum Y-Stück 7 und durch die Fluidführungseinheit 2 und wird vom Patienten Pt mithilfe der patientenseitigen Koppeleinheit 1 eingeatmet.

Eine Fluidfördereinheit, beispielsweise ein Gebläse 4 oder eine Pumpe oder eine Kolben-Zylinder-Einheit, erzeugt einen Volumenfluss (Volumenstrom), beispielsweise einen zeitlich konstanten Volumenfluss, sowie einen Druck, beispielsweise einen zeitlich konstanten Druck. Der zeitlich konstante Druck liegt beispielsweise zwischen 10 mbar und 100 mbar.

Ein erster Drucksensor 5.1 misst den tatsächlichen Druck P_{3.1} im ersten Segment 3.1. Ein optionaler zweiter Drucksensor 5.2 misst den tatsächlichen Druck P_{3.2} im zweiten Segment 3.2. Ein dritter Drucksensor 5.3 misst den Druck am Atemweg (pressure in airway, P_{AW}), bevorzugt an einer Messposition nahe der patientenseitigen Koppeleinheit 1. Ein erster Volumenflusssensor 6.1 misst den tatsächlichen Volumenfluss durch das erste Segment 3.1 hindurch. Ein zweiter Volumenflusssensor 6.2 misst den tatsächlichen Volumenfluss Vol' durch das zweite Segment 3.2 hindurch.

Ein signalverarbeitendes Steuergerät (control unit) 11 erhält jeweils ein Signal von den Sensoren 5.1, 5.2, 5.3 sowie 6.1, 6.2 und steuert eine Ventil-Anordnung 14 mit mindestens einem Ventil, optional mindestens zwei parallel angeordneten Ventilen, an. Die Ventil-Anordnung 14 befindet sich zwischen dem ersten Segment 3.1 und dem zweiten Segment 3.2. Das Steuergerät 11 führt eine Regelung (closed-loop control) mit dem Regelungsziel durch, dass der tatsächliche zeitliche Verlauf des Volumenflusses Vol' durch das zweite Segment 3.2 und / oder des Drucks P_{3.2} im zweiten Segment 3.2 und / oder des Atemwegsdrucks P_{AW} einem vorgegebenen zeitlichen Sollverlauf folgt.

Anmerkung: Wiederholt wird die Formulierung verwendet, dass ein Sensor eine physikalische Größe zu messen vermag. Diese Formulierung bedeutet, dass der Sensor direkt die physikalische Größe zu messen vermag oder mindestens eine andere Größe, die mit der zu messenden Größe korreliert. Die oder eine gemessene andere Größe oder die Kombination der gemessenen anderen Größen zusammen sind daher ein Maß für die zu messende physikalische Größe. Beispielsweise wird eine Druckdifferenz gemessen, und die Druckdifferenz ist ein Maß für einen gesuchten Volumenfluss. Die Messung liefert mindestens einen Wert für die gesuchte physikalische Größe.

Eine Exspirations-Fluidführungseinheit 8, beispielsweise ein weiterer Schlauch, führt vom Y-Stück 7 zurück zum Beatmungsgerät 12. Durch die Exspirations-Fluidführungseinheit 8 fließt das Gas, die der Patient Pt ausgeatmet hat. In der Exspirations-Fluidführungseinheit 8 ist bevorzugt ein end-exspiratorisches Ventil 9 angeordnet, welches sicherstellt, dass in der Lunge des Patienten Pt ein Mindestdruck erhalten bleibt.

In der Regel enthält das vom Patienten Pt ausgeatmete Gas Narkosemittel. Dieses Narkosemittel soll nicht in die Umgebung gelangen. Daher wird mittels der Exspirations-Fluidführungseinheit 8 ein Beatmungskreislauf zwischen dem Beatmungsgerät 12 und der patientenseitigen Koppeleinheit 1 realisiert. Das vom Patienten Pt ausgeatmete Gas wird dank des Beatmungskreislaufs wieder in den Strom des atembaren Gasgemischs, den die Fluidfördereinheit 4 erzeugt, eingespeist.

Aus diesem Beatmungskreislauf muss wenigstens zeitweise ein überschüssiges Gasgemisch Gg abgezweigt werden. Beispielhaft wird ein Überdruckventil 18 gezeigt. Das Überdruckventil 18 öffnet sich, falls der Druck im Beatmungskreislauf oberhalb einer durch die Konstruktion vorgegebenen oberen Druckschranke liegt. Eine stationäre Ansaug-Anordnung 200 saugt überschüssiges Gasgemisch Gg aus dem Beatmungskreislauf ab. Die Ansaug-Anordnung 200 ist hinter der Wand W angeordnet. Das abgesaugte Gasgemisch Gg gelangt in ein stationäres Fluidführungsnetz 40 des Krankenhauses. Das Fluidführungsnetz 40 nimmt bevorzugt Gasgemische von verschiedenen Beatmungsgeräten auf und leitet es in einem Narkosemittel-Aufbereiter oder in die Umgebung.

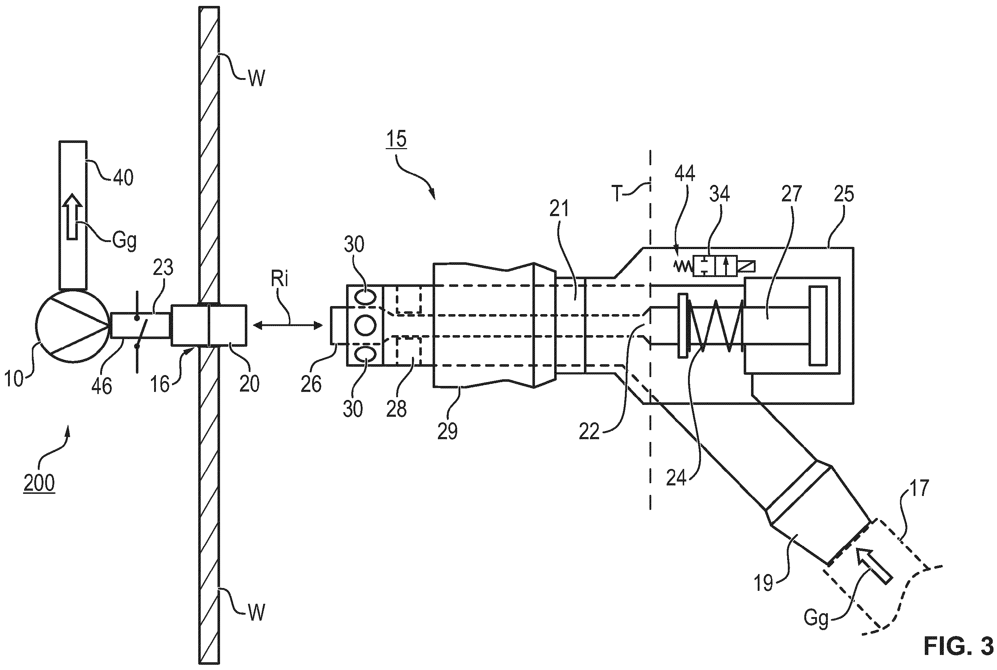

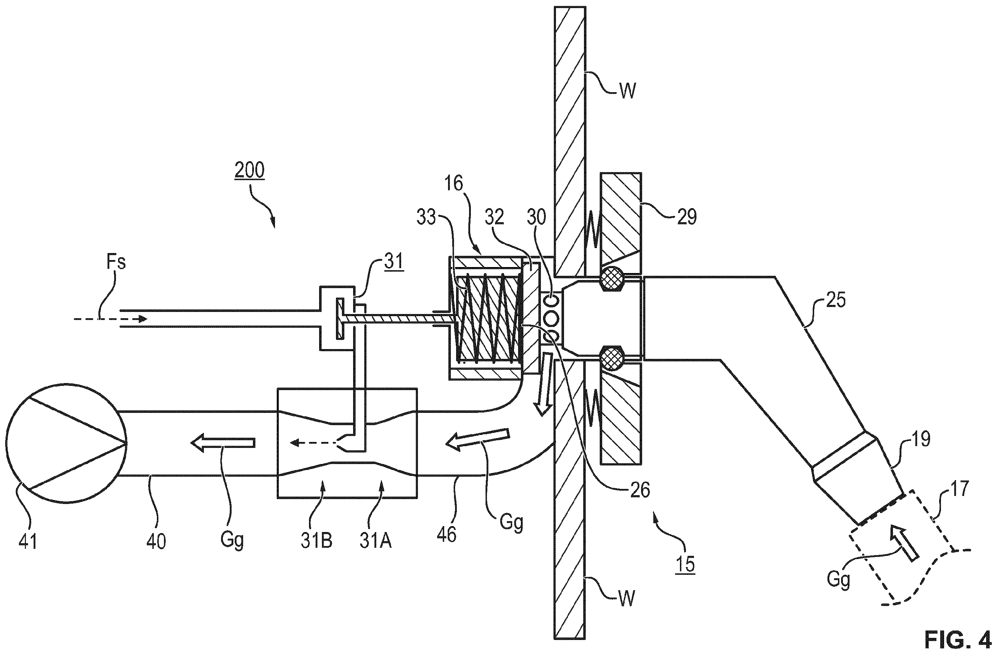

Eine Fluidführungseinheit 35 im Beatmungsgerät 12 führt vom Beatmungskreislauf oder vom Überdruckventil 18 zu einem Anschluss am Gehäuse des Beatmungsgeräts 12. Eine Fluidführungseinheit 17, beispielsweise ein Schlauch, ist mit diesem Anschluss verbunden und leitet das abgezweigte Gasgemisch Gg vom Beatmungsgerät 12 zur Wand W. Am freien Ende der Fluidführungseinheit 17 ist ein Stecker 15 befestigt. Die Fluidführungseinheit 17 lässt sich an einen Schlauchanschluss 19 des Steckers 15 anschließen, vgl. Figur 3 und Figur 4. Der Stecker 15 lässt sich in eine Steckdose 16 in der Wand W einstecken und wieder aus der Steckdose 16 herausziehen. Eine Verriegelung 29, beispielsweise ein Schnappverschluss, hält den eingesteckten Stecker 15 in der Steckdose 16, vgl. Figur 4. Falls der Stecker 15 in die Steckdose 16 eingesteckt ist, so ist eine Fluidverbindung zwischen dem Beatmungsgerät 12 und dem stationären Fluidführungsnetz 40 hergestellt oder lässt sich herstellen. Diese Fluidverbindung führt durch die Fluidführungseinheit 17 und den Stecker 15 und die Steckdose 16 hindurch. Die Ansaug-Anordnung 200 fördert ein Gas durch die Fluidführungseinheit 17 hindurch in das Fluidführungsnetz 40 des Krankenhauses.

Im Ausführungsbeispiel ist das überschüssige Gasgemisch Gg das fortzuleitende Fluid. Das Überdruckventil 18 im Beatmungsgerät 12 gehört zur Quelle für das Fluid Gg, und das Fluidführungsnetz 40 fungiert als die Senke. Die Fluidführungseinheiten 35 und 17 gehören zusammen zur Fluidverbindung zwischen der Quelle und der Senke.

In die Steckdose 16 ist ein Wegeventil 20 oder ein sonstiger Verschluss angeordnet, vgl. Figur 3. Während der Stecker 15 nicht in die Steckdose 16 eingesteckt ist, so ist die Steckdose 16 verschlossen, und durch die Steckdose 16 hindurch wird kein Gas angesaugt und kann kein Gas austreten. Wenn der Stecker 15 in die Steckdose 16 eingeführt wird, wird das Wegeventil 20 geöffnet.

Die Ansaug-Anordnung 200 gehört zu einem Fortleitungssystem für das Gasgemisch. Figur 1 zeigt eine erste Ausgestaltung des Fortleitungssystems, Figur 2 eine zweite Ausgestaltung.

Bei der Ausgestaltung gemäß Figur 1 umfasst die Ansaug-Anordnung 200 eine Fluidfördereinheit 10, wobei die Fluidfördereinheit 10 beispielsweise als Pumpe ausgestaltet ist. Die Fluidfördereinheit 10 lässt sich durch eine entsprechende Ansteuerung einschalten und ausschalten. Eine Leitung 46, z.B. eine Röhre, verbindet die Steckdose 16 mit der Fluidfördereinheit 10. Bei der Ausgestaltung gemäß Figur 2 umfasst die Ansaug-Anordnung 200 eine Unterdruckquelle 41, die bevorzugt dauerhaft eingeschaltet ist und bevorzugt eine eigene Spannungsversorgung aufweist. Die Unterdruckquelle 41 vermag bevorzugt gleichzeitig jeweils ein Gasgemisch von verschiedenen Beatmungsgeräten anzusaugen. Die Leitung 46 führt zum Fluidführungsnetz 40.

Bei der Ausgestaltung gemäß Figur 1 ist die Fluidfördereinheit 10 ausgeschaltet, wenn der Stecker 15 nicht in die Steckdose 16 eingesteckt ist. Der eingeführte Stecker 15 betätigt einen schematisch gezeigten Kontaktschalter 23, vgl. Figur 3. Der betätigte Kontaktschalter 23 schaltet die Fluidfördereinheit 10 ein. Bei der Ausgestaltung gemäß Figur 2 ist die Unterdruckquelle 41 auch dann eingeschaltet, wenn der Stecker 15 nicht eingesteckt und daher die Steckdose 16 verschlossen ist.

Figur 3 und Figur 4 veranschaulichen zwei Realisierungsformen des Steckers 15. In Figur 3 ist links der gestrichelten senkrechten Trennlinie T der Stecker 15 in einer Querschnittsdarstellung gezeigt, und rechts der Trennlinie T sind die innenliegenden Geometrien als verdeckte Kanten gestrichelt gezeigt. Figur 4 zeigt eine schematische Querschnittsdarstellung.

Der Stecker 15 weist ein Gehäuse 25 auf. In das Gehäuse 25 sind mehrere Öffnungen 30 eingelassen. Wenn der Stecker 15 in die Steckdose 16 eingesteckt ist, führt die Fluidverbindung von der Quelle 12, 17 durch die Öffnungen 30, die Steckdose 16 und die Leitung 46 hindurch zur Senke 40.

Im Inneren des Steckers 15 ist ein Hohlraum 21 angeordnet, vgl. Figur 3. Ein Stößel 22 mit einer Spitze 26 ist in diesem Hohlraum 21 dergestalt beweglich gelagert, dass der Stößel 22 relativ zum Rest des Steckers 15 in die beiden entgegengesetzten Richtungen Ri linear vor und zurück bewegt werden kann. Eine Führung 28 führt den Stößel 22 bei seiner linearen Bewegung im Hohlraum 21. Wenn die Spitze 26 des Stößels 22 ausreichend weit aus dem Hohlraum 21 heraus und durch die Steckdose 16 hindurch auf die Fluidfördereinheit 10 zu bewegt ist, so steht die Spitze 26 über das Gehäuse 25 über. Wenn der Stecker 15 eingesteckt ist und die Spitze 26 übersteht, so werden folgende Schritte ausgelöst: Das Wegeventil 20 wird geöffnet, der Kontaktschalter 23 wird betätigt, und die Fluidfördereinheit 10 wird eingeschaltet. Der Stößel 22 fungiert als das Betätigungselement im Sinne einer vorteilhaften Ausgestaltung.

Eine Druckfeder 24 stützt sich am Gehäuse 25 ab und ist bestrebt, die Spitze 26 des Stößels 22 aus dem Hohlraum 21 herauszuschieben, so dass die Spitze 26 vorne über das Gehäuse 25 übersteht. Die Druckfeder 24 kann insbesondere eine mechanische oder pneumatische Druckfeder sein. Möglich ist, dass mehrere Druckfedern parallel angeordnet sind. Die Druckfeder 24 ist bevorzugt als ein rein mechanisches Bauteil ausgelegt und benötigt weder eine Versorgung mit elektrischer Energie noch mit pneumatischem oder hydraulischem Fluid, um den Stößel 22 zu bewegen und zu halten. Die Druckfeder 24 fungiert als das Rückstellelement dieser Ausgestaltung.

Ein Stellglied 27 im Stecker 15 lässt sich durch eine entsprechende Ansteuerung einschalten und ausschalten. Das Stellglied 27 kann insbesondere einen elektrischen Stellantrieb oder einen sonstigen elektrischen Motor oder eine Kolben-Zylinder-Einheit umfassen oder auf andere Weise elektrisch oder pneumatisch oder hydraulisch arbeiten.

In einer Realisierungsform umfasst das Stellglied 27 einen hydraulischen oder pneumatischen Aktuator sowie ein elektrisch ansteuerbares Ventil, beispielsweise ein Schaltventil 34. In einer Ruhestellung verhindert das Schaltventil 34, dass ein pneumatisches oder hydraulisches Fluid zum Aktuator gelangt. Das Stellglied 27 ist dann ausgeschaltet. Als Reaktion auf eine elektrische Ansteuerung öffnet sich das Schaltventil 34, Fluid fließt zum Aktuator des Stellglieds 27, und das Stellglied 27 ist eingeschaltet. Bevorzugt hält die rückstellende Kraft eines Federelements 44 das Schaltventil 34 in der geschlossenen Ruhestellung.

Das Stellglied 27 umfasst in einer anderen Realisierungsform einen Elektromotor und eine Untersetzung, beispielsweise eine Gewindespindel oder eine Zahnstange. Das Stellglied 27 ist dann so ausgestaltet, dass die Druckfeder 24 bei ausgeschaltetem Elektromotor den Stößel 22 zu bewegen vermag und die Untersetzung diese Bewegung nicht hemmt. Der eingeschaltete Elektromotor zieht den Stößel 22 gegen die Rückstellkraft der Druckfeder 24 in das Gehäuse 25.

Das eingeschaltete Stellglied 27 vermag also bei beiden Realisierungsformen den Stößel 22 gegen die rückstellende Kraft der Druckfeder 24 vollständig in den Hohlraum 21 hineinzuziehen, sodass die Spitze 26 nicht mehr über das Gehäuse 25 übersteht. Das Stellglied 27 ist im Ausführungsbeispiel einseitig wirksam, vermag also den Stößel 22 nur in das Gehäuse 25 hineinzuziehen, aber nicht in die entgegengesetzte Richtung zu bewegen. Bei ausgeschaltetem Stellglied 27 verschiebt die Druckfeder 24 so wie gerade beschrieben den Stößel 22 aus dem Gehäuse 25 heraus und hält ihn.

Figur 4 zeigt eine Ausgestaltung der Ansaug-Anordnung 200. In der gezeigten Realisierungsform wird diese Ausgestaltung mit der zweiten Ausgestaltung gemäß Figur 2 kombiniert, also mit einer dauerhaft eingeschalteten Unterdruckquelle 41. Die Ausgestaltung gemäß Figur 4 lässt sich auch mit der ersten Ausgestaltung kombinieren, also mit einer Fluidfördereinheit 10, die sich einschalten und ausschalten lässt.

Ein Druckgas-Ventil 31 weist eine Ansaugseite 31A und eine Ausgabeseite 31B auf, vgl. Figur 4. Die Ansaugseite 31A steht in einer Fluidverbindung mit dem eingesteckten Stecker 15, die Ausgabeseite 31B in einer Fluidverbindung mit dem stationären Fluidführungsnetz 40. Eine Druckfeder 33 ist bestrebt, eine Druckplatte 32 zu verschließen. In der Darstellung gemäß Figur 4 ist die Druckfeder 33 bestrebt, die Druckplatte 32 nach rechts zu verschieben und dadurch die Fluidverbindung zu verschließen. Die Druckfeder 33 und die Druckplatte 32 bilden zusammen ein Rückschlagventil. Wenn der Stecker 15 in die Steckdose 16 eingesteckt ist, so öffnet die Spitze 26 das Rückschlagventil 32, 33. Die Spitze 26 verschiebt hierbei die Druckplatte 32 gegen die Federkraft der Druckfeder 33, und zwar in der Darstellung gemäß Figur 4 nach links. Bei geöffnetem Rückschlagventil 32, 33 erzeugt ein Fluidstrom Fs aufgrund des Venturi-Effekts einen Unterdruck zwischen der Ansaugseite 31A und der Ausgabeseite 31B, und der erzeugte Unterdruck saugt überschüssiges Gasgemisch Gg aus dem eingesteckten Stecker 15 heraus.

Nachfolgend wird die zweite Ausgestaltung gemäß Figur 2 beschrieben. Bei dieser zweiten Ausgestaltung ist die Unterdruckquelle 41 ständig eingeschaltet. Die Fluidführungseinheiten 35 und 17 stellen eine Fluidverbindung vom Beatmungskreislauf im Beatmungsgerät 12 zur Steckdose 16 her. In dieser Fluidverbindung ist mindestens ein Schaltventil angeordnet. Dieses Schaltventil vermag die Fluidverbindung wahlweise freizugeben oder zu versperren. In Figur 2 sind beispielhaft ein erstes Schaltventil 38.1 im Beatmungsgerät 12, nämlich in der Fluidführungseinheit 35, und ein zweites Schaltventil 38.2 in der Fluidführungseinheit 17 dargestellt. Das oder ein Schaltventil kann an einer der gezeigten Positionen oder auch im Stecker 15 angeordnet sein. Bevorzugt ist ein einziges Schaltventil vorhanden.

Das oder jedes Schaltventil 38.1, 38.2 umfasst jeweils ein mechanisches Rückstellelement 39.1, 39.2, beispielsweise eine mechanische oder pneumatische Feder, und ein Stellglied 42.1, 42.2. Das Rückstellelement 39.1, 39.2 ist bestrebt, das Schaltventil 38.1, 38.2 in eine freigebende Position (Förder-Schaltstellung) zu überführen und in der freigebenden Position zu halten. Wenn das oder jedes Schaltventil 38.1, 38.2 in der freigebenden Position ist, ist eine Fluidverbindung zwischen dem Beatmungskreislauf und dem Fluidführungsnetz 40 freigegeben, wobei diese Fluidverbindung durch die Fluidführungseinheit 35, 17 sowie durch den Stecker 15 und die Steckdose 16 hindurch führt.

Weiterhin umfasst das oder jedes Schaltventil 38.1, 38.2 jeweils ein Stellglied 42.1, 42.2. Das Stellglied 42.1, 42.2 lässt sich durch eine entsprechende Ansteuerung von außen aktivieren. Das aktivierte Stellglied 42.1, 42.2 überführt das Schaltventil 38.1, 38.2 gegen die rückstellende Kraft des Rückstellelements 39.1, 39.2 in eine versperrende Position (Ruhe-Schaltstellung). Wenn das oder mindestens ein Schaltventil 38.1, 38.2 in der versperrenden Position ist, ist die oben beschriebene Fluidverbindung unterbrochen, und die Unterdruckquelle 41 vermag kein Gasgemisch Gg aus dem Beatmungskreislauf im Beatmungsgerät 12 anzusaugen.

Sowohl bei der ersten Ausgestaltung gemäß Figur 1 und Figur 3 als auch bei der Ausgestaltung gemäß Figur 2 sind folgende Zustände des Fortleitungssystems möglich:
- Zustand 1: Der Stecker 15 ist nicht in die Steckdose 16 eingesteckt. Dann verschließt das Wegeventil 20 (Figur 3) bzw. das Rückschlagventil 32, 33 (Figur 4) die Steckdose 16, und bei der ersten Ausgestaltung ist die Fluidfördereinheit 10 ausgeschaltet (Figur 3) bzw. das Druckgas-Ventil 31 ist geschlossen (Figur 4).
- Zustand 2: Der Stecker 15 ist in die Steckdose 16 eingesteckt. Bei der ersten Ausgestaltung (Figur 1 und Figur 3) ist das Stellglied 27 ausgeschaltet. In der Realisierungsform gemäß Figur 3 drückt die Druckfeder 24 die Spitze 26 gegen den Kontaktschalter 23, und die Fluidfördereinheit 10 ist eingeschaltet. In der Realisierungsform gemäß Figur 4 öffnet die Spitze 26 das Rückschlagventil 32, 33 und das Druckgas-Ventil 31. Bei der zweiten Ausgestaltung (Figur 2) ist das Schaltventil 38.1, 38.2 in der freigebenden Position, und das Rückstellelement 39.1, 39.2 hält es in der freigebenden Position.

Ein Gasgemisch Gg wird im Zustand 2 bei beiden Ausgestaltungen durch die Fluidführungseinheit 17 und den Stecker 15 hindurch in das Fluidführungsnetz 40 gesaugt.
- Zustand 3: Der Stecker 15 ist in die Steckdose 16 eingesteckt. Bei der ersten Ausgestaltung ist das Stellglied 27 eingeschaltet und zieht den Stößel 22 gegen die Kraft der Druckfeder 24 zurück. Die Spitze 26 berührt nicht mehr den Kontaktschalter 23 bzw. öffnet nicht mehr das Druckgas-Ventil 31, und die Fluidfördereinheit 10 ist ausgeschaltet (Figur 3) bzw. das Druckgas-Ventil 31 ist geschlossen (Figur 4). Bei der zweiten Ausgestaltung hält das Stellglied 42.1, 42.2 das Schaltventil 38.1, 38.2 in der versperrenden Position.
Überschüssiges Gasgemisch Gg wird im Zustand 3 bei beiden Ausgestaltungen nicht abgesaugt.

Im Zustand 1 kann kein Fluid durch die Steckdose 16 hindurch in das Fluidführungsnetz 40 gelangen. Bei der ersten Ausgestaltung ist die Fluidfördereinheit 10 ausgeschaltet.

Im Zustand 2 saugt die Ansaug-Anordnung 200 in beiden Ausgestaltungen das Gasgemisch Gg durch den Stecker 15 in der Steckdose 16 hindurch in das Fluidführungsnetz 40. Im Zustand 2 wird somit verhindert, dass sich im Beatmungsgerät 12 zu viel überschüssiges Gasgemisch Gg ansammelt. Dies ist ein sicherer Zustand.

Im Zustand 3 ist zwar der Stecker 15 in die Steckdose 16 eingesteckt. Jedoch ist bei der ersten Ausgestaltung die Fluidfördereinheit 10 ausgeschaltet, und bei der zweiten Ausgestaltung ist das oder jedes Schaltventil 38.1, 38.2 in der versperrenden Position. Dadurch wird elektrische Energie eingespart. Bei beiden Ausgestaltungen wird die Menge des Gasgemischs Gg, das in das Fluidführungsnetz 40 gelangt, reduziert verglichen mit einer Ansaugung.

Der Zustand 3 darf auftreten, wenn der Stecker 15 in die Steckdose 16 eingesteckt ist, jedoch das Beatmungsgerät 12 aktuell keine künstliche Beatmung durchführt und auch nicht dafür betriebsbereit ist, eine künstliche Beatmung durchzuführen. Beispielsweise wurde nach einem Einsatz des Beatmungsgeräts 12 der Schritt noch nicht durchgeführt oder vergessen, den Stecker 15 aus der Steckdose 16 zu ziehen, oder der Stecker 15 wurde zwar eingesteckt, die künstliche Beatmung aber noch nicht begonnen. In diesem Fall ist es sinnvoll, dass die Ansaug-Anordnung 200 ausgeschaltet ist oder wenigstens kein Gas saugt. Das Stellglied 27 bewirkt diesen gewünschten Effekt.

Das unerwünschte Ereignis kann auftreten, dass eine elektrische oder hydraulische oder pneumatische Versorgung des Stellglieds 27 unterbrochen ist oder das Stellglied 27 ausfällt. In diesem Fall muss weiterhin überschüssiges Gasgemisch Gg abgesaugt werden, und die Ansaug-Anordnung 200 muss weiterarbeiten und darf nicht ausgeschaltet werden.

Falls das Stellglied 27 ausgeschaltet oder ausgefallen ist, schiebt die Druckfeder 24 den Stößel 22 aus dem Gehäuse 25, und die Ansaug-Anordnung 200 wird in den sicheren Zustand 2 überführt. Diese Überführung bedarf nicht eines Eingriffs eines Benutzers und erfordert auch keine elektrische oder pneumatische oder hydraulische Versorgung eines Bauteils. Bevorzugt wird diese Überführung unabhängig davon durchgeführt, ob das Beatmungsgerät 12 betriebsbereit ist oder nicht.

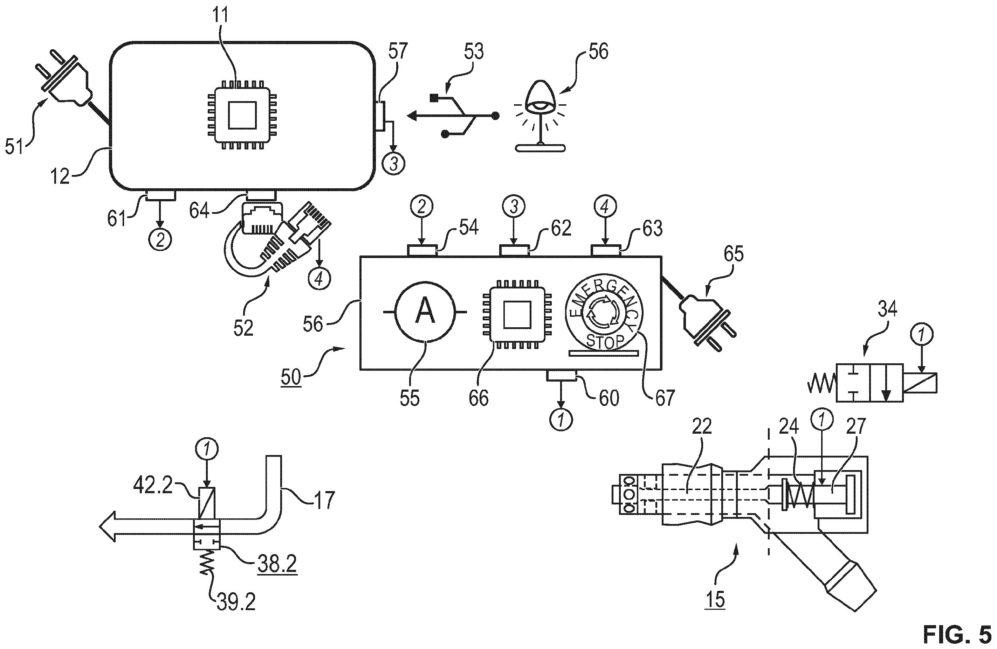

Figur 5 zeigt schematisch einen Netzstecker 51 des Beatmungsgeräts 12 und eine Steuereinheit 50 mit einem eigenen datenverarbeitenden Prozessor 66. Diese Steuereinheit 50 steht in einer Datenverbindung mit dem Stellglied 27 des Steckers 15 oder mit dem Stellglied 42.1, 42.2 des Schaltventils 38.1, 38.2 und vermag das Stellglied 27, 42.1, 42.2 einzuschalten und / oder auszuschalten. Beispielhaft ist gezeigt, dass die Steuereinheit 50 über eine Datenverbindung bewirken kann, das Schaltventil 34 für das Stellglied 27 oder das Stellglied 42.1, 42.2 des Schaltventils 38.1, 38.2 zu öffnen oder zu schließen. Die Steuereinheit 50 umfasst einen Ausgang 60 in Form eines Kommunikations-Ports, über den die Datenverbindung mit dem Schaltventil 34 oder dem Stellglied 42.1, 42.2 hergestellt ist oder hergestellt werden kann. Optional versorgt die Steuereinheit 50 einen Aktuator für das Schaltventil 34 oder das Stellglied 42.1, 42.2 mit elektrischer Energie.

In der gerade beschriebenen Realisierungsform vermag die Steuereinheit 50 das Stellglied 27, 42.1, 42.2 über eine Datenverbindung anzusteuern. Möglich ist auch, dass sich eine Fluidverbindung zwischen der Steuereinheit 50 und dem Stellglied 27, 42.1, 42.2 herstellen lässt und die Steuereinheit 50 über diese Fluidverbindung das Stellglied 27, 42.1, 42.2 mit einem hydraulischen oder pneumatischen Fluid versorgt und dadurch aktiviert.

Bevorzugt umfasst die Steuereinheit 50 weiterhin ein eigenes Gehäuse 56 und ist bevorzugt außerhalb des Beatmungsgeräts 12 angeordnet. Möglich ist, dass das Beatmungsgerät 12 die Steuereinheit 50 mit elektrischer Energie zu versorgen vermag. Bevorzugt hat hingegen die Steuereinheit 50 einen eigenen Netzstecker 65, so dass sich die Steuereinheit 50 unabhängig vom Beatmungsgerät 12 mit elektrischer Energie versorgen lässt. Die Steuereinheit 50 kann zusätzlich oder stattdessen eine eigene Spannungsversorgungseinheit (nicht gezeigt) aufweisen, um unabhängig von einem stationären Spannungsversorgungsnetz zu sein. Möglich ist auch, dass der Netzstecker 51 des Beatmungsgeräts 12 sich in eine korrespondierende Steckdose (nicht gezeigt) im Gehäuse 56 der Steuereinheit 50 oder umgekehrt der Netzstecker 56 sich in eine korrespondierende Steckdose (nicht gezeigt) im Gehäuse des Beatmungsgeräts 12 stecken lässt.

Die Realisierungsform, dass die Steuereinheit 50 ein Gerät mit einem eigenen Gehäuse 56 und bevorzugt mit einer eigenen Spannungsversorgung ist, erleichtert es, ein vorhandenes Fortleitungssystem nachträglich mit einer erfindungsgemäßen Steuereinheit 50 zu versehen, ohne wesentliche Veränderungen am Beatmungsgerät 12 vornehmen zu müssen.

Die Steuereinheit 50 ist bevorzugt wie folgt ausgestaltet: Falls die Steuereinheit 50 nicht mit elektrischer Spannung versorgt wird oder ausgeschaltet oder ausgefallen ist, so ist das Stellglied 27 ausgeschaltet. Dadurch wird ein sicherer Zustand hergestellt.

Das Beatmungsgerät 12 lässt sich mit Hilfe eines üblichen Netzsteckers 51 mit einem stationären Spannungsversorgungsnetz verbinden. Über einen Datenausgang 61 des Beatmungsgeräts 12 und einen Eingang 54 der Steuereinheit 50 vermag die Steuereinheit 50 festzustellen, welche Stromstärke das Beatmungsgerät 12 mithilfe des Netzsteckers 51 aufnimmt. Beispielhaft wird ein Stromstärken-Sensor 55 gezeigt, der mit dem Eingang 54 verbunden ist. Dadurch vermag die Steuereinheit 50 automatisch zu entscheiden, ob das Beatmungsgerät 12 mit dem Spannungsversorgungsnetz verbunden und eingeschaltet ist.

Das Beatmungsgerät 12 vermag im Ausführungsbeispiel eine Beleuchtungseinheit 56 mit elektrischer Spannung zu versorgen. Die Beleuchtungseinheit 56 vermag eine Schreibunterlage oder Anzeigefläche des Beatmungsgeräts 12 zu beleuchten. Die Beleuchtungseinheit 56 ist mit einem USB-Stecker 53 verbunden, der in eine USB-Steckdose 57 gesteckt werden kann. Über diese USB-Verbindung 53, 57 wird die Beleuchtungseinheit 56 mit elektrischer Spannung versorgt. Die Steuereinheit 50 vermag die USB-Steckdose 57 zu überwachen, und zwar mit Hilfe eines Dateneingangs 62. Die Steuereinheit 50 vermag automatisch zu entscheiden, ob über den USB-Stecker 57 ein externes Gerät 56 mit elektrischer Spannung versorgt wird und / oder ob ein elektrischer Strom fließt. Zusätzlich zu der oder anstelle der USB-Steckdose 57 kann die Steuereinheit 50 auch einen Spannungsausgang des Beatmungsgeräts 12 für übliche Wechselspannung oder Gleichspannung überwachen. Wenn das Beatmungsgerät 12 über diesen Spannungsausgang einen elektrischen Verbraucher versorgt, so ist in der Regel das Beatmungsgerät 12 eingeschaltet und führt eine künstliche Beatmung durch oder ist wenigstens dafür bereit, eine künstliche Beatmung durchzuführen.

Zwischen dem Beatmungsgerät 12 und der Steuereinheit 50 lässt sich eine Datenverbindung 52 herstellen, beispielsweise mit Hilfe eines Ethernet-Kabels oder eines kabelgebundenen Datennetzes, z.B. gemäß dem Standard "Service-Oriented Device Connectivity" (SDC), oder drahtlos per Funkwellen. Diese Datenverbindung 52 führt von einem Datenausgang 64 des Beatmungsgeräts 12 zu einem Dateneingang 63 der Steuereinheit 50. In einer Ausgestaltung ist ein Datenkabel für die Datenverbindung 52 mit einem Kabel für den Netzstecker 51 des Beatmungsgeräts 12 verbunden. Diese Ausgestaltung reduziert die Gefahr, dass die Datenverbindung 52 unabsichtlich unterbrochen wird.

Über diese Datenverbindung 52 vermag die Steuereinheit 50 einen aktuellen Betriebszustand des Beatmungsgeräts 12 zu erfassen. Mögliche Betriebszustände sind beispielsweise:
- Das Beatmungsgerät 12 ist in einem Beatmungsmodus und führt aktuell eine künstliche Beatmung des Patienten Pt durch oder ist wenigstens dafür betriebsbereit, eine künstliche Beatmung durchzuführen.
- Das Beatmungsgerät 12 ist eingeschaltet und befindet sich in einem Ruhezustand (standby mode).
- Das Beatmungsgerät 12 ist ausgeschaltet.

In einer Ausgestaltung vermag die Steuereinheit 50 zusätzlich einen Betriebsparameter des Beatmungsgeräts 12 zu ermitteln, beispielsweise den erzeugten Volumenfluss durch das erste Segment 3.1, wobei der erste Volumenflusssensor 6.1 diesen Volumenfluss gemessen hat, oder den erzeugten Atemwegsdruck P_{AW}. Falls der Volumenfluss oder der Atemwegsdruck P_{AW} für einen ausreichend langen Zeitraum unterhalb einer vorgegebenen oberen Schranke bleiben, so ist die künstliche Beatmung noch nicht begonnen oder ist bereits beendet.

Die Steuereinheit 50 vermag durch ein Signal, das über die Datenverbindung 52 übermittelt wird, folgendes Ereignis zu detektieren: Das Beatmungsgerät 12 hat die künstliche Beatmung des Patienten Pt beendet. In diesem Fall wird das Beatmungsgerät 12 aus dem Beatmungsmodus zunächst in den Ruhezustand überführt und dann ausgeschaltet. Möglich ist auch, dass über die oben beschriebene Datenverbindung 52 explizit eine Nachricht übermittelt wird, welche einen Ausschaltvorgang für das Beatmungsgerät 12 spezifiziert. Die Steuereinheit 50 empfängt und verarbeitet diese Nachricht.

Außerdem vermag die Steuereinheit 50 das Ereignis zu detektieren, dass die Datenverbindung 52 plötzlich unterbrochen wird, beispielsweise weil versehentlich die Ethernet-Verbindung unterbrochen wurde, z. B. weil das Ethernet-Kabel gezogen wurde. In diesem Fall tritt nicht zuvor ein Ruhezustand auf. Möglich ist auch, dass die Datenverbindung 52 laufend darauf überwacht wird, ob sie noch aufrechterhalten wird (Alive Protocol).

Weiter oben wurden ein Zustand 2 und ein Zustand 3 beschrieben. Im Zustand 2 ist das Stellglied 27 ausgeschaltet, die Ansaug-Anordnung 200 ist eingeschaltet, und zwischen dem Beatmungsgerät 12 und dem Fluidführungsnetz 40 ist eine Fluidverbindung hergestellt, die durch die Fluidführungseinheit 17 und den Stecker 15 führt. Überschüssiges Gasgemisch Gg wird abgesaugt. Im Zustand 3 ist das Stellglied 27, 42.1, 42.2 eingeschaltet, und das eingeschaltete Stellglied 27, 42.1, 42.2 bewirkt, dass die Ansaug-Anordnung 200 ausgeschaltet oder die Fluidverbindung unterbrochen und daher kein überschüssiges Gasgemisch Gg abgesaugt und somit Energie eingespart wird.

Die nachfolgende Beschreibung bezieht sich auf die Situation, dass der Stecker 15 in die Steckdose 16 eingesteckt ist. Idealerweise tritt bei eingestecktem Stecker 15 der Zustand 2 dann auf, während das Beatmungsgerät 12 eine künstliche Beatmung durchführt oder wenigstens dafür betriebsbereit ist, und ansonsten der Zustand 3. Sichergestellt werden muss, dass der Zustand 2 mit Sicherheit dann hergestellt ist, während das Beatmungsgerät 12 eine künstliche Beatmung durchführt oder wenigstens dafür betriebsbereit ist. Falls das Beatmungsgerät 12 keine künstliche Beatmung durchführt und auch nicht betriebsbereit ist, sollte der Zustand 3 hergestellt sein. Falls dann aber stattdessen der Zustand 2 hergestellt ist, so wird zwar elektrische und / oder pneumatische Energie verbraucht, es tritt aber keine sicherheitskritische Situation auf. Dieser sichere Zustand wird zum einen durch folgende Funktion erreicht: Das Rückstellelement 24, 39.1, 39.2 bewirkt, dass der Zustand 2 hergestellt ist, wenn das Stellglied 27, 38.1, 38.2 oder die Steuereinheit 50 ausgefallen oder ausgeschaltet sind. Diese Funktion stellt sicher, dass der Zustand 2 mindestens bei einer künstlichen Beatmung hergestellt ist, aber nur relativ selten, idealerweise überhaupt nicht, wenn das Beatmungsgerät 12 keine künstliche Beatmung durchführt.

Nachfolgend wird beschrieben, wie mit Hilfe der Steuereinheit 50 eine sicherheitskritische Situation vermieden wird und trotzdem elektrische Energie eingespart wird. Die Steuereinheit 50 wendet eine vorgegebene rechnerauswertbare Entscheidungslogik an. Die Entscheidungslogik ist eine logische Verknüpfung des Auftretens oder Nicht-Auftretens von mehreren vorgegebenen möglichen Ereignissen und legt fest, ob das Stellglied 27, 42.1, 42.2 eingeschaltet (Zustand 3) oder ausgeschaltet (Zustand 2) ist.

Die Steuereinheit 50 vermag für jedes mögliche Ereignis, das in die Entscheidungslogik einfließt, automatisch zu entscheiden, ob dieses mögliche Ereignis aktuell tatsächlich eingetreten ist oder nicht. Im Ausführungsbeispiel sind dies die folgenden möglichen Ereignisse:
- Das Beatmungsgerät 12 führt eine künstliche Beatmung durch oder wird darauf vorbereitet, eine künstliche Beatmung durchzuführen. Daher verbraucht das Beatmungsgerät 12 Strom, wobei die Stromstärke oberhalb einer vorgegebenen unteren Stromstärken-Schranke liegt. Dies wird beispielsweise mithilfe des Stromstärken-Sensors 55 detektiert. Dann muss der Zustand 2 vorliegen.
- Das Beatmungsgerät 12 hat die künstliche Beatmung beendet. Über die Datenverbindung 52 ermittelt die Steuereinheit 50, dass das Beatmungsgerät 12 vom Beatmungsmodus in den Ruhezustand überführt und dann ausgeschaltet wird. Jetzt kann der Zustand 3 hergestellt werden.
- Die künstliche Beatmung wurde noch nicht begonnen oder ist beendet. Die Steuereinheit 50 stellt fest, dass der Volumenstrom durch das erste Segment 3.1 oder der Atemwegsdruck P_{AW} für einen ausreichend langen Zeitraum kleiner als eine vorgegebene obere Schranke sind. Auch dann kann der Zustand 3 hergestellt werden.
- Über die oben beschriebene Datenverbindung 52 wird eine Nachricht übermittelt. Diese Nachricht umfasst die Information, dass das Beatmungsgerät 12 sich in einem Ruhezustand (einem Standby-Modus) befindet. Auch dann kann der Zustand 3 hergestellt werden.
- An das Beatmungsgerät 12 ist ein elektrischer Verbraucher angeschlossen. Beispielsweise ist die Lichtquelle 56 mit Hilfe des USB-Steckers 53 mit der USB-Steckdose 57 verbunden. Dann sollte der Zustand 2 vorliegen, es sei denn, die Steuereinheit 50 hat positiv das Ereignis detektiert, dass das Beatmungsgerät 12 in einem Ruhezustand ist.
- Die Datenverbindung 52 ist unterbrochen, ohne dass zuvor ein Ruhezustand detektiert und ohne dass eine Nachricht mit einem bevorstehenden Ausschalten übermittelt wurde. Dann sollte der Zustand 2 vorliegen.

In einer Ausgestaltung umfasst die Steuereinheit 50 zusätzlich einen Notausschalter 67. Falls ein Benutzer den Notausschalter 67 betätigt, so wird die Steuereinheit 50 ausgeschaltet, und dadurch wird auch das Stellglied 27 ausgeschaltet, und der Zustand 2 wird hergestellt.

**Bezugszeichenliste**

| | |
|---|---|
| 1 | patientenseitige Koppeleinheit in Form einer Atemmaske, mit der Fluidführungseinheit 2 und mit dem Patienten Pt verbunden |
| 2 | Fluidführungseinheit, verbindet das Y-Stück 7 mit der patientenseitigen Koppeleinheit 1 |
| 3.1 | erstes Segment der Inspirations-Fluidführungseinheit, führt von der Fluidfördereinheit 4 zur Ventil-Anordnung 14 |
| 3.2 | zweites Segment der Inspirations-Fluidführungseinheit, führt von der Ventil-Anordnung 14 zum Y-Stück 7 |
| 4 | Fluidfördereinheit in Form eines Gebläses, stößt ein Gasgemisch in das erste Segment 3.1 hinein aus, ist mit dem Versorgungsanschluss 13 verbunden |
| 5.1 | erster Drucksensor, misst den tatsächlichen Druck P_{3.1} im ersten Segment 3.1, wobei der Druck P_{3.1} in der Regel von der Fluidfördereinheit 4 erzeugt wird |
| 5.2 | zweiter Drucksensor, misst den tatsächlichen Druck P_{3.2} im zweiten Segment 3.2 |
| 5.3 | dritter Drucksensor, misst den tatsächlichen Atemwegsdruck P_{AW} |
| 6.1 | erster Volumenflusssensor, misst den tatsächlichen Volumenfluss durch das erste Segment 3.1 hindurch |
| 6.2 | zweiter Volumenflusssensor, misst den tatsächlichen Volumenfluss Vol' durch das zweite Segment 3.2 hindurch |
| 7 | Y-Stück, verbindet die Fluidführungseinheiten 3.2 und 8 auf der einen Seite mit der Fluidführungseinheit 2 auf der anderen Seite |
| 8 | Exspirations-Fluidführungseinheit, führt vom Y-Stück 7 zurück zum Beatmungsgerät 12 |
| 9 | end-exspiratorisches Ventil in der Exspirations-Fluidführungseinheit 8 |
| 10 | Fluidfördereinheit der Aufnahme-Anordnung 200, lässt sich einschalten und abschalten, vermag das Gasgemisch Gg in das Fluidführungsnetz 40 einzusaugen |
| 11 | signalverarbeitendes Steuergerät, empfängt und verarbeitet Einstell-Parameter-Signale von den Sensoren 5.1, 5.2 sowie 6.1, 6.2, steuert die Ventil-Anordnung 14 und in der ersten Ausgestaltung das Schaltventil 34 und in der zweiten Ausgestaltung die Schaltventile 38.1, 38.2 an |
| 12 | Beatmungsgerät, umfasst die Fluidfördereinheit 4, das Steuergerät 11, die Ventil-Anordnung 14, die Stellglied-Anordnung 20, die Sensoren 5.1, 5.2 und 6.1, 6.2 und den Versorgungsanschluss 13 |
| 13 | Versorgungsanschluss des Beatmungsgeräts 12, mit der Fluidfördereinheit 4 verbunden |
| 14 | Ventil-Anordnung, zwischen den Segmenten 3.1 und 3.2 angeordnet |
| 15 | Stecker am freien Ende der Fluidführungseinheit 17, umfasst in einer Ausgestaltung das Gehäuse 25, den Stößel 22, die Druckfeder 24, das Stellglied 27, die Verriegelung 29 und das Schaltventil 34 |
| 16 | Steckdose in der Wand W, nimmt den Stecker 15 auf |
| 17 | Fluidführungseinheit, führt von der Fluidführungseinheit 35 zum Stecker 15, mit dem Schlauchanschluss 19 verbunden oder verbindbar, wird in einer Ausgestaltung vom Schaltventil 38.2 wahlweise freigegeben oder verschlossen |
| 18 | Überdruckventil im Beatmungskreislauf |
| 19 | Schlauchanschluss am Stecker 15, lässt sich mit der Fluidführungseinheit 17 verbinden |
| 20 | Absperrventil in der Steckdose 16, lässt sich vom Stecker 15 öffnen |
| 21 | Hohlraum im Inneren des Steckers 15, nimmt den Stößel 22 auf |
| 22 | Stößel im Hohlraum 21, kann in die beiden entgegengesetzten Richtungen Ri linear bewegt werden, hat die Spitze 26 |
| 23 | Kontaktschalter in der Steckdose 16 |
| 24 | Druckfeder im Stecker 15, stützt sich am Gehäuse 25 ab, ist bestrebt, die Spitze 26 des Stößels 22 aus dem Hohlraum 21 herauszuschieben |
| 25 | Gehäuse des Steckers 15 |
| 26 | Spitze des Stößels 22, vermag das Absperrventil 20 und das Druckgas-Ventil 31 zu öffnen |
| 27 | Stellglied im Stecker 15, ist bestrebt, den Stößel 22 gegen die Kraft der Druckfeder 24 in den Hohlraum 21 hineinzuziehen, wird von der Steuereinheit 50 eingeschaltet und ausgeschaltet |
| 28 | Führung im Hohlraum 21 für den Stößel 22 |
| 29 | Verriegelung für den Stecker 15 |
| 30 | Öffnungen im Gehäuse 25 |
| 31 | Druckgas-Ventil, weist die Ansaugseite 31A und die Ausgabeseite 31B auf |
| 31A | Ansaugseite des Druckgas-Ventils 31 |
| 31B | Ausgabeseite des Druckgas-Ventils 31 |
| 32 | Kontaktschalter in Form einer Druckplatte |
| 33 | Druckfeder für die Druckplatte 32 |
| 34 | ansteuerbares Schaltventil, welches den Fluss eines Fluids zum Stellglied 27 freigibt oder unterbricht |
| 35 | Fluidführungseinheit im Beatmungsgerät 12, verbindet den Beatmungskreislauf mit der Fluidführungseinheit 17 |
| 36 | Narkosemittelverdunster |
| 38.1, 38.2 | ansteuerbares Schaltventil, welches eine Fluidverbindung zwischen den Beatmungsgerät 12 und der Steckdose 16 freigibt oder unterbricht, umfasst das Rückstellelement 39.1, 39.2 und das Stellglied 42.1, 42.2 |
| 39.1, 39.2 | mechanisches Rückstellelement für das Schaltventil 38.1, 38.2 |
| 40 | stationäres Fluidführungsnetz des Krankenhauses, nimmt abgesaugtes überschüssiges Gasgemisch Gg auf |
| 41 | Unterdruckquelle, ist dauerhaft eingeschaltet, saugt das Gasgemisch Gg in das Fluidführungsnetz 40 |
| 42.1, 42.2 | ansteuerbares Stellglied des Schaltventils 38.1, 38.2 |
| 44 | Federelement, hält das Schaltventil 34 in der geschlossenen Stellung |
| 46 | Leitung von der Steckdose zum Fluidführungsnetz 40 |
| 50 | signalverarbeitende Steuereinheit, umfasst das Gehäuse 56, 10 Prozessor 66, den Netzstecker 65, den Notausschalter 67, die Dateneingänge 54, 62, 63 und den Datenausgang 60, schaltet bei der ersten Ausgestaltung das Stellglied 27 und bei der zweiten Ausgestaltung das Stellglied 42.1, 42.2 ein und aus |
| 51 | Netzstecker des Beatmungsgeräts 12 |
| 52 | Datenverbindung zwischen dem Beatmungsgerät 12 und der Steuereinheit 50, wird über den Datenausgang 54 und den Dateneingang 63 hergestellt |
| 53 | USB-Stecker der Beleuchtungseinheit 56, lässt sich in die USB-Steckdose 57 einstecken |
| 54 | Dateneingang der Steuereinheit 50, mit dem Stromstärken-Sensor 55 verbunden |
| 55 | Stromstärken-Sensor, mit dem Eingang 54 verbunden |
| 56 | Beleuchtungseinheit für eine Schreibunterlage des Beatmungsgeräts 12, mit dem USB-Stecker 53 verbunden |
| 57 | USB-Steckdose, in die sich der USB-Stecker 53 einstecken lässt |
| 58 | Gehäuse der Steuereinheit 50 |
| 60 | Datenausgang in Form eines Kommunikations-Ports, über den die Datenverbindung mit dem Schaltventil 34 oder dem Stellglied 42.1, 42.2 hergestellt ist |
| 61 | Datenausgang des Beatmungsgeräts 12, wird für die Überwachung der USB-Steckdose 57 verwendet |
| 62 | Dateneingang der Steuereinheit 50, wird für die Überwachung der USB-Steckdose 57 verwendet |
| 63 | Dateneingang der Steuereinheit 50, wird für die Datenverbindung 52 verwendet |
| 64 | Datenausgang des Beatmungsgeräts 12, wird für die Datenverbindung 52 verwendet |
| 65 | Netzstecker der Steuereinheit 50 |
| 66 | Prozessor der Steuereinheit 50 |
| 67 | Notausschalter der Steuereinheit 50 |
| 100 | Beatmungs-Anordnung, umfasst das Beatmungsgerät 12, die Fluidführungseinheiten 2, 3.1, 3.2, 8 das Y-Stück 7 und die patientenseitige Koppeleinheit 1 |
| 200 | Ansaug-Anordnung in der Wand W, umfasst in einer Ausgestaltung die Fluidfördereinheit 10 und in einer anderen Ausgestaltung das Druckgas-Ventil 30 |
| Fs | Fluidstrom, der Gasgemisch Gg absaugt |
| Gg | überschüssiges Gasgemisch, wird aus dem Beatmungskreislauf abgezweigt und durch den Stecker 15 hindurch in die Fluidführungsnetz 40 gefördert, fungiert als das Fluid |
| P_{AW} | Druck im zweiten Segment 3.2, vom Drucksensor 5.2 gemessen |
| Pt | Patient, wird künstlich beatmet, mit der patientenseitigen Koppeleinheit 1 verbunden |
| Ri | entgegengesetzte Richtungen, in die der Stößel 22 bewegt werden kann |
| T | Trennlinie |
| Vol' | Volumenfluss durch das zweite Segment 3.2, vom Volumenflusssensor 6.2 gemessen |
| W | Wand, hinter der sich die Absaug-Anordnung 200 und das Fluidführungsnetz 40 befinden, weist die Steckdose 16 auf |

## Patentansprüche

1. Fortleitungssystem für ein Fluid (Gg),
wobei das Fortleitungssystem
- eine Ansaug-Anordnung (200),
- ein Schaltelement (23, 32, 38.1, 38.2),
- ein Rückstellelement (24, 39.1, 39.2) für das Schaltelement (23, 32, 38.1, 38.2),
- ein einschaltbares und ausschaltbares Stellglied (27, 42.1, 42.2),
- eine signalverarbeitende Steuereinheit (50),
- einen Stecker (15) und
- eine Steckdose (16)
umfasst,
wobei der Stecker (15) sich in die Steckdose (16) stecken lässt,
wobei bei eingestecktem Stecker (15) eine Fluidverbindung (35, 17) zwischen einer Quelle (18) und einer Senke (40) für das Fluid (Gg) hergestellt oder herstellbar ist,
wobei die Ansaug-Anordnung (200) dazu ausgestaltet ist, Fluid (Gg) von der Quelle (18) zur Senke (40) zu fördern, insbesondere durch Ansaugung,
wobei das Schaltelement (23, 32, 38.1, 38.2)
- in eine Förder-Schaltstellung und in eine Ruhe-Schaltstellung schaltbar ist und
- dazu ausgestaltet ist, in der Förder-Schaltstellung das Fortleitungssystem in einen Förderzustand und in der Ruhe-Schaltstellung das Fortleitungssystem in einen Ruhezustand zu versetzen,
wobei das Fortleitungssystem dazu ausgestaltet ist, im Förderzustand das Fluid (Gg) mit Hilfe der Ansaug-Anordnung (200) zur Senke (40) zu fördern,
wobei das Fördern von Fluid im Ruhezustand des Fortleitungssystems unterbrochen und / oder unterbunden ist,
wobei das Rückstellelement (24, 39.1, 39.2)
- als mechanisches Bauteil ausgestaltet ist,
- einen Ruhezustand aufweist und
- bei einer Auslenkung aus dem Ruhezustand eine rückstellende Kraft ausübt,
wobei das Rückstellelement (24, 39.1, 39.2) im Ruhezustand das Schaltelement (23, 32, 38.1, 38.2) in der Förder-Schaltstellung hält und die rückstellende Kraft bestrebt ist, das Schaltelement (23, 32, 38.1, 38.2) in die Förder-Schaltstellung zu schalten,
wobei das Stellglied (27, 42.1, 42.2) dazu ausgestaltet ist,
- nach einem Einschalten das Schaltelement (23, 32, 38.1, 38.2) gegen die rückstellende Kraft des Rückstellelements (24, 39.1, 39.2) in die Ruhe-Schaltstellung zu schalten, und
- nach einem Ausschalten zu ermöglichen, dass das Rückstellelement (24, 39.1, 39.2) das Schaltelement (23, 32, 38.1, 38.2) in die Förder-Schaltstellung schaltet,
wobei die Steuereinheit (50) dazu ausgestaltet ist,
- das Fortleitungssystem und / oder die Quelle (18) auf mindestens ein Ereignis zu überwachen, bei dem aus der Quelle (18) kein Fluid (Gg) austritt und
- dann, wenn die Steuereinheit (50) positiv das oder mindestens ein Ereignis detektiert hat, bei dem aus der Quelle (18) kein Fluid (Gg) austritt, das Stellglied (27, 42.1, 42.2) einzuschalten, und
wobei das Fortleitungssystem so ausgestaltet ist, dass
mindestens dann, wenn die Steuereinheit (50) nicht positiv detektiert hat, dass aus der Quelle (18) kein Fluid (Gg) austritt,
das Stellglied (27, 42.1, 42.2) ausgeschaltet ist.

2. Fortleitungssystem nach Anspruch 1,
**dadurch gekennzeichnet, dass**
die Steuereinheit (50) dazu ausgestaltet ist, als ein Ereignis, bei dem aus der Quelle (18) kein Fluid (Gg) austritt,
positiv das Ereignis zu detektieren, dass die Quelle (18) oder ein Gerät (12), welches die Quelle (18) umfasst, ausgeschaltet ist.

3. Fortleitungssystem nach einem der vorhergehenden Ansprüche,
**dadurch gekennzeichnet, dass**
die Steuereinheit (50) dazu ausgestaltet ist, mindestens ein Indiz dafür zu detektieren, dass aus der Quelle (18) Fluid (Gg) austritt und / oder die Quelle (18) dazu betriebsbereit ist, Fluid (Gg) auszustoßen,
wobei die Steuereinheit (50) weiterhin dazu ausgestaltet ist, dann, wenn die Steuereinheit (50) mindestens ein solches Indiz detektiert hat,
zu bewirken oder sicherzustellen, dass das Stellglied (27, 42.1, 42.2) ausgeschaltet wird oder ist.

4. Fortleitungssystem nach Anspruch 3,
**dadurch gekennzeichnet, dass**
die Steuereinheit (50) dazu ausgestaltet ist, als Indiz dafür, dass aus der Quelle (18) Fluid (Gg) austritt und / oder die Quelle (18) dazu betriebsbereit ist, Fluid (Gg) auszustoßen,
das Ereignis zu detektieren, dass die Quelle (18) elektrische Energie aufnimmt oder verbraucht oder abgibt,
wobei die detektierte Energieaufnahme oder der Energieverbrauch oder die Energieabgabe durch die Quelle (18) oberhalb einer vorgegebenen unteren Schranke liegt.

5. Fortleitungssystem nach einem der vorhergehenden Ansprüche,
**dadurch gekennzeichnet, dass**
die Steuereinheit (50) dazu ausgestaltet ist,
- ein Indiz dafür zu detektieren, dass eine Datenverbindung (52) zwischen der Quelle (18) und der Steuereinheit (50) unterbrochen ist, und
- dann, wenn ein solches Indiz detektiert ist, zu bewirken oder sicherzustellen, dass das Stellglied (27, 42.1, 42.2) ausgeschaltet wird oder ist.

6. Fortleitungssystem nach einem der vorhergehenden Ansprüche,
**dadurch gekennzeichnet, dass**
die Ansaug-Anordnung (200) sich einschalten und ausschalten lässt,
der Stecker (15) ein beweglich gelagertes Betätigungselement (22) umfasst und
das Rückstellelement (24, 39.1, 39.2) und das Stellglied (27, 42.1, 42.2) Bestandteile des Steckers (15) sind,
wobei das Fortleitungssystem
- im Förderzustand ist, wenn die Ansaug-Anordnung (200) eingeschaltet ist, und
- im Ruhezustand ist, wenn die Ansaug-Anordnung (200) ausgeschaltet ist,
wobei das Schaltelement (23, 32, 38.1, 38.2) dazu ausgestaltet ist, abhängig von seiner Schaltstellung die Ansaug-Anordnung (200) im eingeschalteten oder im ausgeschalteten Zustand zu halten, und
wobei das Betätigungselement (22)
- in eine Betätigungsposition bewegbar ist und
- in der Betätigungsposition das Schaltelement (23, 32, 38.1, 38.2) derart betätigt, dass die Ansaug-Anordnung (200) im eingeschalteten Zustand ist oder bleibt.

7. Fortleitungssystem nach Anspruch 6,
**dadurch gekennzeichnet, dass**
das Rückstellelement (24, 39.1, 39.2) im Ruhezustand das Betätigungselement (22) in der Betätigungsposition und dadurch die Ansaug-Anordnung (200) im eingeschalteten Zustand hält und
das eingeschaltete Stellglied (27, 42.1, 42.2)
- das Betätigungselement (22) gegen die rückstellende Kraft des Rückstellelements (24, 39.1, 39.2) aus der Betätigungsposition heraus bewegt und
- dadurch bewirkt, dass die Ansaug-Anordnung (200) ausgeschaltet wird.

8. Fortleitungssystem nach einem der vorhergehenden Ansprüche,
**dadurch gekennzeichnet, dass**
das Schaltelement (23, 32, 38.1, 38.2) mindestens ein Schaltventil (38.1, 38.2) umfasst,
wobei das Schaltventil (38.1, 38.2) in der Fluidverbindung (35, 17), die von der Quelle (18) zur Senke (40) führt, angeordnet ist,
wobei das Schaltventil (38.1, 38.2) in eine Förder-Schaltstellung und in eine Ruhe-Schaltstellung schaltbar ist,
wobei das Schaltventil (38.1, 38.2)
- in der Förder-Schaltstellung die Fluidverbindung (35, 17) freigibt und
- in der Ruhe-Schaltstellung die Fluidverbindung (35, 17) unterbricht,
wobei das Rückstellelement (24, 39.1, 39.2) im Ruhezustand das Schaltventil (38.1, 38.2) in der Förder-Schaltstellung hält und
wobei das eingeschaltete Stellglied (27, 42.1, 42.2) dazu ausgestaltet ist, nach einem Einschalten das Schaltventil (38.1, 38.2) gegen die rückstellende Kraft des Rückstellelements (24, 39.1, 39.2) in die Ruhe-Schaltstellung zu schalten.

9. Fortleitungssystem nach einem der vorhergehenden Ansprüche,
**dadurch gekennzeichnet, dass**
die Quelle (18) ein Bestandteil eines Geräts (12) ist und
die Steuereinheit (50) ein eigenes Gehäuse (56) umfasst und räumlich beabstandet vom Gerät (12) mit der Quelle (18) angeordnet ist.

10. System umfassend
- ein Fortleitungssystem nach einem der vorhergehenden Ansprüche,
- eine Quelle (18) für ein Fluid (Gg) und
- eine Senke (40) für das Fluid (Gg),
wobei bei eingestecktem Stecker (15) eine Fluidverbindung (35, 17) zwischen der Quelle (18) und der Senke (40) hergestellt oder herstellbar ist und
wobei diese Fluidverbindung (35, 17) bei nicht eingestecktem Stecker (15) unterbrochen ist.

11. System nach Anspruch 10,
**dadurch gekennzeichnet, dass**
das System ein Beatmungsgerät (12) umfasst und
die Senke eine Aufnahme-Anordnung (40) für das Fluid (Gg) umfasst,
wobei das Beatmungsgerät (12) zur künstlichen Beatmung eines Patienten (Pt) ausgestaltet ist,
wobei die Quelle (18) ein Bestandteil des Beatmungsgeräts (12) ist,
wobei während der künstlichen Beatmung im und / oder am Körper des Patienten (Pt) eine patientenseitige Koppeleinheit (1) angeordnet ist,
wobei während der künstlichen Beatmung wenigstens zeitweise eine Fluidverbindung zwischen dem Beatmungsgerät (12) und der patientenseitigen Koppeleinheit (1) hergestellt ist und
wobei das Fluid (Gg) aus dem Beatmungsgerät (12) und / oder der Fluidverbindung zwischen dem Beatmungsgerät (12) und der patientenseitigen Koppeleinheit (1) stammt.

12. System nach Anspruch 11,
**dadurch gekennzeichnet, dass**
während der künstlichen Beatmung ein Beatmungskreislauf zwischen dem Beatmungsgerät (12) und der patientenseitigen Koppeleinheit (1) hergestellt ist,
wobei das Fluid (Gg) aus dem Beatmungskreislauf stammt und mindestens ein Narkosemittel und / oder ein Medikament aufweist.

13. Verfahren zum Fördern eines Fluids (Gg) von einer Quelle (18) zu einer Senke (40),
wobei das Verfahren unter Verwendung eines Fortleitungssystems durchgeführt wird,
wobei das Fortleitungssystem
- eine Ansaug-Anordnung (200),
- ein Schaltelement (23, 32, 38.1, 38.2),
- ein Rückstellelement (24, 39.1, 39.2) für das Schaltelement (23, 32, 38.1, 38.2),
- eine signalverarbeitende Steuereinheit (50),
- ein einschaltbares und ausschaltbares Stellglied (27, 42.1, 42.2),
- einen Stecker (15) und
- eine Steckdose (16)
umfasst,
wobei das Rückstellelement (24, 39.1, 39.2)
- als mechanisches Bauteil ausgestaltet ist und
- einen Ruhezustand aufweist,
wobei das Verfahren die Schritte umfasst, dass
- der Stecker (15) in die Steckdose (16) gesteckt wird oder ist,
- das Schaltelement (23, 32, 38.1, 38.2) in eine Förder-Schaltstellung geschaltet wird oder ist und
- das Schaltelement (23, 32, 38.1, 38.2) in der Förder-Schaltstellung das Fortleitungssystem in einen Förderzustand versetzt,
wobei im Förderzustand die weiteren Schritte durchgeführt werden, dass
- eine Fluidverbindung (35, 17) zwischen der Quelle (18) und der Senke (40) für das Fluid hergestellt wird oder ist,
- die Ansaug-Anordnung das Fluid (Gg) durch die Fluidverbindung (35, 17) hindurch von der Quelle (18) zur Senke (40) fördert,
- das Stellglied (27, 42.1, 42.2) ausgeschaltet ist,
- das Rückstellelement (24, 39.1, 39.2) im Ruhezustand das Schaltelement (23, 32, 38.1, 38.2) in der Förder-Schaltstellung hält,
- das Rückstellelement (24, 39.1, 39.2) bei einer Auslenkung aus dem Ruhezustand eine rückstellende Kraft ausübt,
- die rückstellende Kraft bestrebt ist, das Schaltelement (23, 32, 38.1, 38.2) in die Förder-Schaltstellung zu schalten und
- die Steuereinheit (50) das Fortleitungssystem und / oder die Quelle (18) auf mindestens ein Ereignis überwacht, bei dem aus der Quelle (18) kein Fluid (Gg) austritt,
wobei dann, wenn bei eingestecktem Stecker (15) die Steuereinheit (50) positiv das oder mindestens ein Ereignis detektiert hat, bei dem aus der Quelle (18) kein Fluid (Gg) austritt,
die weiteren Schritte durchgeführt werden, dass
- die Steuereinheit (50) das Stellglied (27, 42.1, 42.2) einschaltet,
- das eingeschaltete Stellglied (27, 42.1, 42.2) das Schaltelement (23, 32, 38.1, 38.2) gegen die rückstellende Kraft des Rückstellelements (24, 39.1, 39.2) in die Ruhe-Schaltstellung schaltet,
- das Schaltelement (23, 32, 38.1, 38.2) in der Ruhe-Schaltstellung bewirkt, dass das Fortleitungssystem in einen Ruhezustand versetzt wird und
- im Ruhezustand des Fortleitungssystems das Fördern von Fluid (Gg) durch die Fluidverbindung (35, 17) hindurch unterbrochen und / oder unterbunden ist.

14. Verfahren nach Anspruch 13,
**dadurch gekennzeichnet, dass**
die Ansaug-Anordnung (200) sich einschalten und ausschalten lässt,
der Stecker (15) ein beweglich gelagertes Betätigungselement (22) umfasst und
das Rückstellelement (24, 39.1, 39.2) und das Stellglied (27, 42.1, 42.2) Bestandteile des Steckers (15) sind,
wobei das Fortleitungssystem
- im Förderzustand ist, wenn die Ansaug-Anordnung (200) eingeschaltet ist, und
- im Ruhezustand ist, wenn die Ansaug-Anordnung (200) ausgeschaltet ist,
wobei dann, wenn das Fortleitungssystem im Förderzustand ist, die weiteren Schritte durchgeführt werden, dass
- das Rückstellelement (24, 39.1, 39.2) im Ruhezustand das Betätigungselement (22) in einer Betätigungsposition hält,
- das Betätigungselement (22) in der Betätigungsposition das Schaltelement (23, 32, 38.1, 38.2) betätigt,
- das betätigte Schaltelement (23, 32, 38.1, 38.2) die Ansaug-Anordnung (200) in den eingeschalteten Zustand versetzt und / oder im eingeschalteten Zustand hält, und
wobei der Schritt, dass das eingeschaltete Stellglied (27, 42.1, 42.2) das Schaltelement (23, 32, 38.1, 38.2) in die Ruhe-Schaltstellung schaltet, den Schritt umfasst, dass
- das eingeschaltete Stellglied (27, 42.1, 42.2) das Betätigungselement (22) gegen die rückstellende Kraft des Rückstellelements (24, 39.1, 39.2) aus der Betätigungsposition heraus bewegt und
- dadurch bewirkt wird, dass die Ansaug-Anordnung (200) ausgeschaltet wird.

15. Verfahren nach Anspruch 13 oder Anspruch 14,
**dadurch gekennzeichnet, dass**
das Schaltelement (23, 32, 38.1, 38.2) mindestens ein Schaltventil (38.1, 38.2) umfasst,
wobei das Schaltventil (38.1, 38.2) in der Fluidverbindung (35, 17), die von der Quelle (18) zur Senke (40) führt, angeordnet ist,
wobei dann, wenn das Fortleitungssystem im Förderzustand ist, die weiteren Schritte durchgeführt werden, dass
- das Rückstellelement (24, 39.1, 39.2) im Ruhezustand das Schaltventil (38.1, 38.2) in eine Förder-Schaltstellung schaltet und / oder in der Förder-Schaltstellung hält und
- das Schaltventil (38.1, 38.2) in der Förder-Schaltstellung die Fluidverbindung (35, 17) freigibt und
wobei der Schritt, dass das eingeschaltete Stellglied (27, 42.1, 42.2) das Schaltelement (23, 32, 38.1, 38.2) in die Ruhe-Schaltstellung schaltet, den Schritt umfasst, dass
- das eingeschaltete Stellglied (27, 42.1, 42.2) das Schaltventil (38.1, 38.2) gegen die rückstellende Kraft des Rückstellelements (24, 39.1, 39.2) in die Ruhe-Schaltstellung schaltet und
- dadurch bewirkt wird, dass das Fortleitungssystem in den Ruhezustand überführt wird.

## Geänderte Patentansprüche

### Geänderte Patentansprüche gemäss Regel 137(2) EPÜ.

1. Fortleitungssystem für ein Fluid (Gg),
wobei das Fortleitungssystem
- eine Ansaug-Anordnung (200),
- ein Schaltelement (23, 32, 38.1, 38.2),
- ein Rückstellelement (24, 39.1, 39.2) für das Schaltelement (23, 32, 38.1, 38.2),
- ein einschaltbares und ausschaltbares Stellglied (27, 42.1, 42.2),
- eine signalverarbeitende Steuereinheit (50),
- einen Stecker (15) und
- eine Steckdose (16)
umfasst,
wobei der Stecker (15) sich in die Steckdose (16) stecken lässt,
wobei bei eingestecktem Stecker (15) eine Fluidverbindung (35, 17) zwischen einer Quelle (18) und einer Senke (40) für das Fluid (Gg) hergestellt oder herstellbar ist,
wobei die Ansaug-Anordnung (200) dazu ausgestaltet ist, Fluid (Gg) von der Quelle (18) zur Senke (40) zu fördern, insbesondere durch Ansaugung,
wobei das Schaltelement (23, 32, 38.1, 38.2)
- in eine Förder-Schaltstellung und in eine Ruhe-Schaltstellung schaltbar ist und
- dazu ausgestaltet ist, in der Förder-Schaltstellung das Fortleitungssystem in einen Förderzustand und in der Ruhe-Schaltstellung das Fortleitungssystem in einen Ruhezustand zu versetzen,
wobei das Fortleitungssystem dazu ausgestaltet ist, im Förderzustand das Fluid (Gg) mit Hilfe der Ansaug-Anordnung (200) zur Senke (40) zu fördern,
wobei das Fördern von Fluid im Ruhezustand des Fortleitungssystems unterbrochen und / oder unterbunden ist,
wobei das Rückstellelement (24, 39.1, 39.2)
- als mechanisches Bauteil ausgestaltet ist,
- einen Ruhezustand aufweist und
- bei einer Auslenkung aus dem Ruhezustand eine rückstellende Kraft ausübt,
wobei das Rückstellelement (24, 39.1, 39.2) im Ruhezustand das Schaltelement (23, 32, 38.1, 38.2) in der Förder-Schaltstellung hält und die rückstellende Kraft bestrebt ist, das Schaltelement (23, 32, 38.1, 38.2) in die Förder-Schaltstellung zu schalten,
wobei das Stellglied (27, 42.1, 42.2) dazu ausgestaltet ist,
- nach einem Einschalten das Schaltelement (23, 32, 38.1, 38.2) gegen die rückstellende Kraft des Rückstellelements (24, 39.1, 39.2) in die Ruhe-Schaltstellung zu schalten und
- nach einem Ausschalten zu ermöglichen, dass das Rückstellelement (24, 39.1, 39.2) das Schaltelement (23, 32, 38.1, 38.2) wieder in die Förder-Schaltstellung schaltet,
wobei die Steuereinheit (50) dazu ausgestaltet ist,
- das Fortleitungssystem und / oder die Quelle (18) auf mindestens ein Ereignis zu überwachen, bei dem aus der Quelle (18) kein Fluid (Gg) austritt und
- dann, wenn die Steuereinheit (50) positiv das oder mindestens ein Ereignis detektiert hat, bei dem aus der Quelle (18) kein Fluid (Gg) austritt, das Stellglied (27, 42.1, 42.2) einzuschalten, und
wobei die Steuereinheit (50) dazu ausgestaltet ist,
- mindestens ein Indiz dafür zu detektieren, dass aus der Quelle (18) Fluid (Gg) austritt und / oder die Quelle (18) dazu betriebsbereit ist, Fluid (Gg) auszustoßen, und
- dann, wenn die Steuereinheit (50) mindestens ein solches Indiz detektiert hat,
zu bewirken oder sicherzustellen, dass das Stellglied (27, 42.1, 42.2) ausgeschaltet wird oder ist, und
wobei das Fortleitungssystem so ausgestaltet ist, dass
mindestens dann, wenn die Steuereinheit (50) nicht positiv detektiert hat, dass aus der Quelle (18) kein Fluid (Gg) austritt,
das Stellglied (27, 42.1, 42.2) ausgeschaltet ist.

2. Fortleitungssystem nach Anspruch 1,
**dadurch gekennzeichnet, dass**
die Steuereinheit (50) dazu ausgestaltet ist, als ein Ereignis, bei dem aus der Quelle (18) kein Fluid (Gg) austritt,
positiv das Ereignis zu detektieren, dass die Quelle (18) oder ein Gerät (12), welches die Quelle (18) umfasst, ausgeschaltet ist.

3. Fortleitungssystem nach einem der vorhergehenden Ansprüche,
**dadurch gekennzeichnet, dass**
die Steuereinheit (50) dazu ausgestaltet ist, als Indiz dafür, dass aus der Quelle (18) Fluid (Gg) austritt und / oder die Quelle (18) dazu betriebsbereit ist, Fluid (Gg) auszustoßen,
das Ereignis zu detektieren, dass die Quelle (18) elektrische Energie aufnimmt oder verbraucht oder abgibt,
wobei die detektierte Energieaufnahme oder der Energieverbrauch oder die Energieabgabe durch die Quelle (18) oberhalb einer vorgegebenen unteren Schranke liegt.

4. Fortleitungssystem nach einem der vorhergehenden Ansprüche,
**dadurch gekennzeichnet, dass**
die Steuereinheit (50) dazu ausgestaltet ist,
- ein Indiz dafür zu detektieren, dass eine Datenverbindung (52) zwischen der Quelle (18) und der Steuereinheit (50) unterbrochen ist, und
- dann, wenn ein solches Indiz detektiert ist, zu bewirken oder sicherzustellen, dass das Stellglied (27, 42.1, 42.2) ausgeschaltet wird oder ist.

5. Fortleitungssystem nach einem der vorhergehenden Ansprüche,
**dadurch gekennzeichnet, dass**
die Ansaug-Anordnung (200) sich einschalten und ausschalten lässt,
der Stecker (15) ein beweglich gelagertes Betätigungselement (22) umfasst und
das Rückstellelement (24, 39.1, 39.2) und das Stellglied (27, 42.1, 42.2) Bestandteile des Steckers (15) sind,
wobei das Fortleitungssystem
- im Förderzustand ist, wenn die Ansaug-Anordnung (200) eingeschaltet ist, und
- im Ruhezustand ist, wenn die Ansaug-Anordnung (200) ausgeschaltet ist,
wobei das Schaltelement (23, 32, 38.1, 38.2) dazu ausgestaltet ist, abhängig von seiner Schaltstellung die Ansaug-Anordnung (200) im eingeschalteten oder im ausgeschalteten Zustand zu halten, und
wobei das Betätigungselement (22)
- in eine Betätigungsposition bewegbar ist und
- in der Betätigungsposition das Schaltelement (23, 32, 38.1, 38.2) derart betätigt, dass die Ansaug-Anordnung (200) im eingeschalteten Zustand ist oder bleibt.

6. Fortleitungssystem nach Anspruch 5,
**dadurch gekennzeichnet, dass**
das Rückstellelement (24, 39.1, 39.2) im Ruhezustand das Betätigungselement (22) in der Betätigungsposition und dadurch die Ansaug-Anordnung (200) im eingeschalteten Zustand hält und
das eingeschaltete Stellglied (27, 42.1, 42.2)
- das Betätigungselement (22) gegen die rückstellende Kraft des Rückstellelements (24, 39.1, 39.2) aus der Betätigungsposition heraus bewegt und
- dadurch bewirkt, dass die Ansaug-Anordnung (200) ausgeschaltet wird.

7. Fortleitungssystem nach einem der vorhergehenden Ansprüche,
**dadurch gekennzeichnet, dass**
das Schaltelement (23, 32, 38.1, 38.2) mindestens ein Schaltventil (38.1, 38.2) umfasst,
wobei das Schaltventil (38.1, 38.2) in der Fluidverbindung (35, 17), die von der Quelle (18) zur Senke (40) führt, angeordnet ist,
wobei das Schaltventil (38.1, 38.2) in eine Förder-Schaltstellung und in eine Ruhe-Schaltstellung schaltbar ist,
wobei das Schaltventil (38.1, 38.2)
- in der Förder-Schaltstellung die Fluidverbindung (35, 17) freigibt und
- in der Ruhe-Schaltstellung die Fluidverbindung (35, 17) unterbricht, wobei das Rückstellelement (24, 39.1, 39.2) im Ruhezustand das Schaltventil (38.1, 38.2) in der Förder-Schaltstellung hält und
wobei das eingeschaltete Stellglied (27, 42.1, 42.2) dazu ausgestaltet ist, nach einem Einschalten das Schaltventil (38.1, 38.2) gegen die rückstellende Kraft des Rückstellelements (24, 39.1, 39.2) in die Ruhe-Schaltstellung zu schalten.

8. Fortleitungssystem nach einem der vorhergehenden Ansprüche,
**dadurch gekennzeichnet, dass**
die Quelle (18) ein Bestandteil eines Geräts (12) ist und
die Steuereinheit (50) ein eigenes Gehäuse (56) umfasst und räumlich beabstandet vom Gerät (12) mit der Quelle (18) angeordnet ist.

9. System umfassend
- ein Fortleitungssystem nach einem der vorhergehenden Ansprüche,
- eine Quelle (18) für ein Fluid (Gg) und
- eine Senke (40) für das Fluid (Gg),
wobei bei eingestecktem Stecker (15) eine Fluidverbindung (35, 17) zwischen der Quelle (18) und der Senke (40) hergestellt oder herstellbar ist und
wobei diese Fluidverbindung (35, 17) bei nicht eingestecktem Stecker (15) unterbrochen ist.

10. System nach Anspruch 9,
**dadurch gekennzeichnet, dass**
das System ein Beatmungsgerät (12) umfasst und
die Senke eine Aufnahme-Anordnung (40) für das Fluid (Gg) umfasst,
wobei das Beatmungsgerät (12) zur künstlichen Beatmung eines Patienten (Pt) ausgestaltet ist,
wobei die Quelle (18) ein Bestandteil des Beatmungsgeräts (12) ist,
wobei während der künstlichen Beatmung im und / oder am Körper des Patienten (Pt) eine patientenseitige Koppeleinheit (1) angeordnet ist,
wobei während der künstlichen Beatmung wenigstens zeitweise eine Fluidverbindung zwischen dem Beatmungsgerät (12) und der patientenseitigen Koppeleinheit (1) hergestellt ist und
wobei das Fluid (Gg) aus dem Beatmungsgerät (12) und / oder der Fluidverbindung zwischen dem Beatmungsgerät (12) und der patientenseitigen Koppeleinheit (1) stammt.

11. System nach Anspruch 10,
**dadurch gekennzeichnet, dass**
während der künstlichen Beatmung ein Beatmungskreislauf zwischen dem Beatmungsgerät (12) und der patientenseitigen Koppeleinheit (1) hergestellt ist,
wobei das Fluid (Gg) aus dem Beatmungskreislauf stammt und mindestens ein Narkosemittel und / oder ein Medikament aufweist.

12. Verfahren zum Fördern eines Fluids (Gg) von einer Quelle (18) zu einer Senke (40),
wobei das Verfahren unter Verwendung eines Fortleitungssystems durchgeführt wird,
wobei das Fortleitungssystem
- eine Ansaug-Anordnung (200),
- ein Schaltelement (23, 32, 38.1, 38.2),
- ein Rückstellelement (24, 39.1, 39.2) für das Schaltelement (23, 32, 38.1, 38.2),
- eine signalverarbeitende Steuereinheit (50),
- ein einschaltbares und ausschaltbares Stellglied (27, 42.1, 42.2),
- einen Stecker (15) und
- eine Steckdose (16)
umfasst,
wobei das Rückstellelement (24, 39.1, 39.2)
- als mechanisches Bauteil ausgestaltet ist und
- einen Ruhezustand aufweist,
wobei das Verfahren die Schritte umfasst, dass
- der Stecker (15) in die Steckdose (16) gesteckt wird oder ist,
- das Schaltelement (23, 32, 38.1, 38.2) in eine Förder-Schaltstellung geschaltet wird oder ist und
- das Schaltelement (23, 32, 38.1, 38.2) in der Förder-Schaltstellung das Fortleitungssystem in einen Förderzustand versetzt,
wobei im Förderzustand die weiteren Schritte durchgeführt werden, dass
- eine Fluidverbindung (35, 17) zwischen der Quelle (18) und der Senke (40) für das Fluid hergestellt wird oder ist,
- die Ansaug-Anordnung das Fluid (Gg) durch die Fluidverbindung (35, 17) hindurch von der Quelle (18) zur Senke (40) fördert,
- das Stellglied (27, 42.1, 42.2) ausgeschaltet ist,
- das Rückstellelement (24, 39.1, 39.2) im Ruhezustand das Schaltelement (23, 32, 38.1, 38.2) in der Förder-Schaltstellung hält,
- das Rückstellelement (24, 39.1, 39.2) bei einer Auslenkung aus dem Ruhezustand eine rückstellende Kraft ausübt,
- die rückstellende Kraft bestrebt ist, das Schaltelement (23, 32, 38.1, 38.2) wieder in die Förder-Schaltstellung zu schalten und
- die Steuereinheit (50) das Fortleitungssystem und / oder die Quelle (18) auf mindestens ein Ereignis überwacht, bei dem aus der Quelle (18) kein Fluid (Gg) austritt,
wobei dann, wenn bei eingestecktem Stecker (15) die Steuereinheit (50) positiv das oder mindestens ein Ereignis detektiert hat, bei dem aus der Quelle (18) kein Fluid (Gg) austritt,
die weiteren Schritte durchgeführt werden, dass
- die Steuereinheit (50) das Stellglied (27, 42.1, 42.2) einschaltet,
- das eingeschaltete Stellglied (27, 42.1, 42.2) das Schaltelement (23, 32, 38.1, 38.2) gegen die rückstellende Kraft des Rückstellelements (24, 39.1, 39.2) in eine Ruhe-Schaltstellung schaltet,
- das Schaltelement (23, 32, 38.1, 38.2) in der Ruhe-Schaltstellung bewirkt, dass das Fortleitungssystem in einen Ruhezustand versetzt wird und
- im Ruhezustand des Fortleitungssystems das Fördern von Fluid (Gg) durch die Fluidverbindung (35, 17) hindurch unterbrochen und / oder unterbunden ist.
wobei die Steuereinheit (50) dazu ausgestaltet ist, mindestens ein Indiz dafür zu detektieren, dass aus der Quelle (18) Fluid (Gg) austritt und / oder die Quelle (18) dazu betriebsbereit ist, Fluid (Gg) auszustoßen,
wobei der weitere Schritt durchgeführt wird, dass
dann, wenn die Steuereinheit (50) mindestens ein solches Indiz detektiert hat, die Steuereinheit (50) bewirkt oder sicherstellt, dass das Stellglied (27, 42.1, 42.2) ausgeschaltet wird oder ist, und
wobei mindestens dann, wenn die Steuereinheit (50) nicht positiv detektiert hat, dass aus der Quelle (18) kein Fluid (Gg) austritt,
die Steuereinheit (50) bewirkt, dass das Stellglied (27, 42.1, 42.2) ausgeschaltet wird oder ausgeschaltet ist.

13. Verfahren nach Anspruch 12,
**dadurch gekennzeichnet, dass**
die Ansaug-Anordnung (200) sich einschalten und ausschalten lässt,
der Stecker (15) ein beweglich gelagertes Betätigungselement (22) umfasst und
das Rückstellelement (24, 39.1, 39.2) und das Stellglied (27, 42.1, 42.2) Bestandteile des Steckers (15) sind,
wobei das Fortleitungssystem
- im Förderzustand ist, wenn die Ansaug-Anordnung (200) eingeschaltet ist, und
- im Ruhezustand ist, wenn die Ansaug-Anordnung (200) ausgeschaltet ist,
wobei dann, wenn das Fortleitungssystem im Förderzustand ist, die weiteren Schritte durchgeführt werden, dass
- das Rückstellelement (24, 39.1, 39.2) im Ruhezustand das Betätigungselement (22) in einer Betätigungsposition hält,
- das Betätigungselement (22) in der Betätigungsposition das Schaltelement (23, 32, 38.1, 38.2) betätigt,
- das betätigte Schaltelement (23, 32, 38.1, 38.2) die Ansaug-Anordnung (200) in den eingeschalteten Zustand versetzt und / oder im eingeschalteten Zustand hält, und
wobei der Schritt, dass das eingeschaltete Stellglied (27, 42.1, 42.2) das Schaltelement (23, 32, 38.1, 38.2) in die Ruhe-Schaltstellung schaltet, den Schritt umfasst, dass
- das eingeschaltete Stellglied (27, 42.1, 42.2) das Betätigungselement (22) gegen die rückstellende Kraft des Rückstellelements (24, 39.1, 39.2) aus der Betätigungsposition heraus bewegt und
- dadurch bewirkt wird, dass die Ansaug-Anordnung (200) ausgeschaltet wird.

14. Verfahren nach Anspruch 12 oder Anspruch 13,
**dadurch gekennzeichnet, dass**
das Schaltelement (23, 32, 38.1, 38.2) mindestens ein Schaltventil (38.1, 38.2) umfasst,
wobei das Schaltventil (38.1, 38.2) in der Fluidverbindung (35, 17), die von der Quelle (18) zur Senke (40) führt, angeordnet ist,
wobei dann, wenn das Fortleitungssystem im Förderzustand ist, die weiteren Schritte durchgeführt werden, dass
- das Rückstellelement (24, 39.1, 39.2) im Ruhezustand das Schaltventil (38.1, 38.2) in eine Förder-Schaltstellung schaltet und / oder in der Förder-Schaltstellung hält und
- das Schaltventil (38.1, 38.2) in der Förder-Schaltstellung die
Fluidverbindung (35, 17) freigibt und
wobei der Schritt, dass das eingeschaltete Stellglied (27, 42.1, 42.2) das Schaltelement (23, 32, 38.1, 38.2) in die Ruhe-Schaltstellung schaltet, den Schritt umfasst, dass
- das eingeschaltete Stellglied (27, 42.1, 42.2) das Schaltventil (38.1, 38.2) gegen die rückstellende Kraft des Rückstellelements (24, 39.1, 39.2) in die Ruhe-Schaltstellung schaltet und
- dadurch bewirkt wird, dass das Fortleitungssystem in den Ruhezustand überführt wird.
